# EUROPEAN PATENT SPECIFICATION

(11) **EP 2 904 391 B1**
(45) Date of publication and mention of the grant of the patent: **17.01.2018**
(21) Application number: 13773237.6
(22) Date of filing: 04.10.2013
(51) Int. Cl.: G01N 33/50

(54) **MITOCHONDRIAL TOXICITY TEST**
TEST EINER MITOCHONDRIALEN TOXIZITÄT
ESSAI DE TOXICITÉ MITOCHONDRIALE

(30) Priority: 05.10.2012 DK 201270609
(43) Date of publication of application: 12.08.2015
(73) Proprietor: Neurovive Pharmaceutical AB, 223 81 Lund (SE)
(72) Inventor: SJÖVALL, Fredrik, S-226 53 Lund (SE); EHINGER, Johannes, S-223 58 Lund (SE); HANSSON, Magnus, S-261 33 Landskrona (SE); ELMER, Eskil, S-224 58 Lund (SE); BATCHELLER, Derek Gregory, B-1000 Brussels (BE)
(74) Representative: Chas. Hude A/S
(86) International application number: PCT/EP2013/070666
(87) International publication number: WO 2014/053617

(56) References cited:
- WO-A1-2010/019041
- WO-A1-2012/075591
- WO-A2-2011/150221
- SJÖVALL FREDRIK ET AL: "Temporal increase of platelet mitochondrial respiration is negatively associated with clinical outcome in patients with sepsis.", CRITICAL CARE (LONDON, ENGLAND) 2010, vol. 14, no. 6, R214, 2010, pages 1-11, XP002689874, ISSN: 1466-609X
- BEESON C C ET AL: "A high-throughput respirometric assay for mitochondrial biogenesis and toxicity", ANALYTICAL BIOCHEMISTRY, ACADEMIC PRESS INC, NEW YORK, vol. 404, no. 1, 1 September 2010 (2010-09-01), pages 75-81, XP027104737, ISSN: 0003-2697 [retrieved on 2010-05-11]
- BRENNER-LAVIE HANIT ET AL: "Dopamine modulates mitochondrial function in viable SH-SY5Y cells possibly via its interaction with complex I: relevance to dopamine pathology in schizophrenia.", BIOCHIMICA ET BIOPHYSICA ACTA FEB 2008, vol. 1777, no. 2, February 2008 (2008-02), pages 173-185, XP002689875, ISSN: 0006-3002

## Description

### Field of the invention

The present invention provides a novel method that is useful in drug screening. In particular the method is useful for testing effects of substances on the mitochondria, notably toxic or beneficial effects of drug substances or candidate drug substances. The method is based on measurement in live human mitochondria *ex vivo,* but in a setting as near the *in vivo* situation as possible. The method is also useful for testing substances impact on the mitochondrial respiration.

### Background of the invention

In drug development it is difficult to estimate if a drug will affect the energy-producing mitochondria when used in humans. In recent years many of the new drugs approved by the FDA have later been withdrawn due to toxic effects like cardio- or hepatotoxicity. Thus, of new drugs that were registered by the FDA between 1994 and 2006, 38 drugs were withdrawn due to toxic effect on the liver and heart, which are typical symptoms of mitochondrial toxicity. Evidently, these new drugs passed the standard toxicity test performed before approval, and the toxic effects were only observed after a large number of patients had been treated with the drugs. Dykens & Will (2007) point out that evidence indicates that mitochondrial dysfunction played a role in the toxicity of several drug substances that were withdrawn from the market and only reintroduced with severe restrictions.

Moreover, in recent years mitochondrial function has received much interest and it is generally believed that dysfunction of mitochondria is a contributing factor to the pathogenesis of a large number of diseases (e.g. muscle disorders, neuropathies, cardiomyopathies, encephalopathy, sepsis-induced multiple organ failure).

Currently, mitochondrial toxicity of drug candidates is mostly evaluated in animals and cell cultures, results which are difficult to translate to future human use.

Beeson et al. (Analytical Biochemistry 2010, 404 (1), pp. 75-81) describe a cell-based assay for testing the toxic effects of drugs on mitochondria.

Sjovall et al. (Critical Care 2010, 14 (6), pp. 1-11) describe analysis of mitochondrial dysfunction is platelets of individuals suffering from sepsis.

Accordingly, there is a need for developing a test for mitochondrial toxicity, which is sensitive, reproducible, reliable and easy, and which can be used in drug development testing.

### Detailed description of the invention

The present invention relates to a method involving human blood components containing mitochondria. The advantage of the developed method is measurement of drug toxicity (or beneficial effects) in live human mitochondria *ex vivo,* but in as *near in vivo* situation as possible. Human blood components containing mitochondria (white blood cells and/or platelets) are investigated in plasma, which provides a very close relation to the *in vivo* situation. Thus all buffering capacity, serum albumin, electrolytes, hydrolysing enzymes etc. are present during the measurements.

The novel method will directly result in an estimation of a realistic toxic blood concentration in humans that is not possible in cell cultures or animal studies, which are the current methods used. Thus, candidate drug substances can be tested and screened for human toxicity at a much earlier stage than is now possible. In turn, this will permit the elimination of toxic drug candidates at an earlier stage, thus obviating much animal testing and future human suffering. It will facilitate the selection of candidate drug substances at an early stage, thus reducing the overall time and cost of drug development.

The method can be used in different setting with variations in the set-up depending on the purpose of the method.

Thus, without limiting the scope thereto, the method according to the present invention can be used to:
1. Screening and selection of early or late stage drug candidates in cells derived from blood from healthy individuals or in so-called buffy coat, which is a concentrated solution of platelets and white blood cells.
2. Testing a patient's sensitivity to a known mitochondrial toxicant
3. Analysing mitochondrial drug toxicity in clinical trials
4. Analysing beneficial effects of drugs intended to improve mitochondrial function

A suitable method for screening and selection of potential drug candidates or for testing a person's sensitivity to a substance with effect on mitochondria (i.e. items 1 and 2 above) is a method comprising
i) subjecting a sample of human blood cells containing mitochondria to high-resolution respirometry
ii) adding to the cell sample a substance that increases the leakage of the inner mitochondrial membrane to protons,
iii) adding to the sample from ii) a test substance in a vehicle,
vi) adding to the sample from iii) an inhibitor of mitochondrial complex I-function,
v) adding to the sample from vi) an inhibitor of mitochondrial complex III-function, and
vi) comparing the mitochondrial oxygen consumption before and after addition of the test substance with the mitochondrial oxygen consumption of the vehicle, wherein a decrease in mitochondrial oxygen consumption induced by the test substance indicates a negative effect on the mitochondria, and wherein an increase in mitochondrial oxygen consumption induced by the test substance indicates a positive effect on mitochondria.

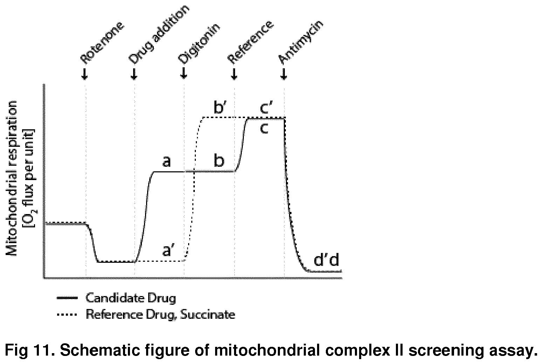

An alternative protocol for screening and selecting potential drug candidates or for testing the sensitivity of a person to a substance with effect on mitochondria (i.e. items 1 and 2 above) is to investigate the effect of such a test substance in a complex II screening assay. Such a method is illustrated in Figure 11 and comprises
i) subjecting a sample of cells containing live mitochondria isolated from a human blood sample to high-resolution respirometry,
ii) contacting the sample of cell to a substance that inhibits complex I respiration,
iii) adding a test sample containing a test substance in a vehicle,
iv) adding a substance that permeabilizes the plasma membrane,
v) adding a reference substance to the sample obtained in iv), and
v) adding a substance that inhibits complex III respiration.

The results from the test are compared with the results obtained by the method, wherein the test substance used is identical to the reference substance. A typical result is shown in Figure 11, which shows that the candidate drug is able to a certain extent to permeate the plasma membrane (i.e. an increase in respiration is achieved). Addition of digitonin, which makes the plasma membrane permeable, does not result in an further increase in respiration, i.e. no further effect from the test substance. When the reference substance (a complex II-linked substrate such as succinate) is added, which is typically an endogeneous substrate like a succinate, an increase in respiration is observed and maximal capacity is achieved. Then a complex III, such as antimycin A, is added to determine any non-mitochondrial oxygen-consuming activity, such as auto-oxidation of said sample.

Compared with the results from the reference substance, which is an endogeneous substrate such as a succinate, it is seen that the reference substance does not permeate the plasma membrane. Only when the membrane is permeabilized e.g. with digitonin, the respiration increases. Further addition of the endogeneous substance does not change the respiration whereas addition of complex III inhibitor stops the mitochondrial respiration.

Obviously, the pattern observed with a test substance may differ from the one shown in Figure 11. The method gives valuable information regarding whether a test substance can permeate the plasma membrane and influence the mitochondrial oxygen-consuming activity in a positive manner (i.e. increase in complex II mitochondrial respiration). Accordingly, the most ideal test substance will have a level a which is higher than a' and where level a is or approaches level b'. More details are given in the examples herein.

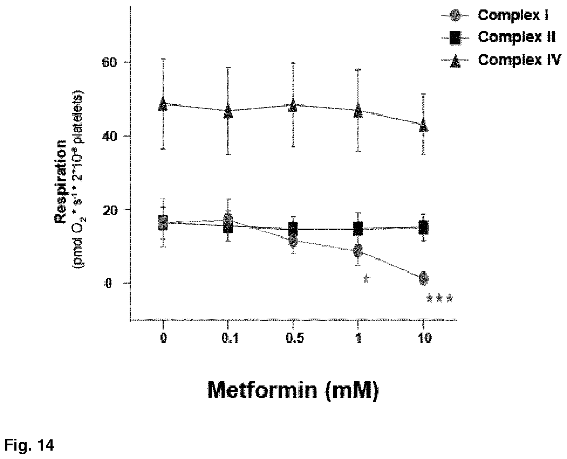

Another approach is to study the influence of a test substance on complex I, complex II and/or complex IV respiration. By studying complexes I, II and/or IV respiration more details can be obtained with respect to the impact of a specific substance on the mitochondrial respiration and to evaluate any positive or negative effect on the mitochondrial function. Figure 14 shows the influence of increasing concentration of metformin on complexes I, II and IV mitochondrial respiration. The figure shows specific dysfunction of complex I respiration with increasing concentrations of metformin, whereas complex II and IV respiration seem to be unaffected.

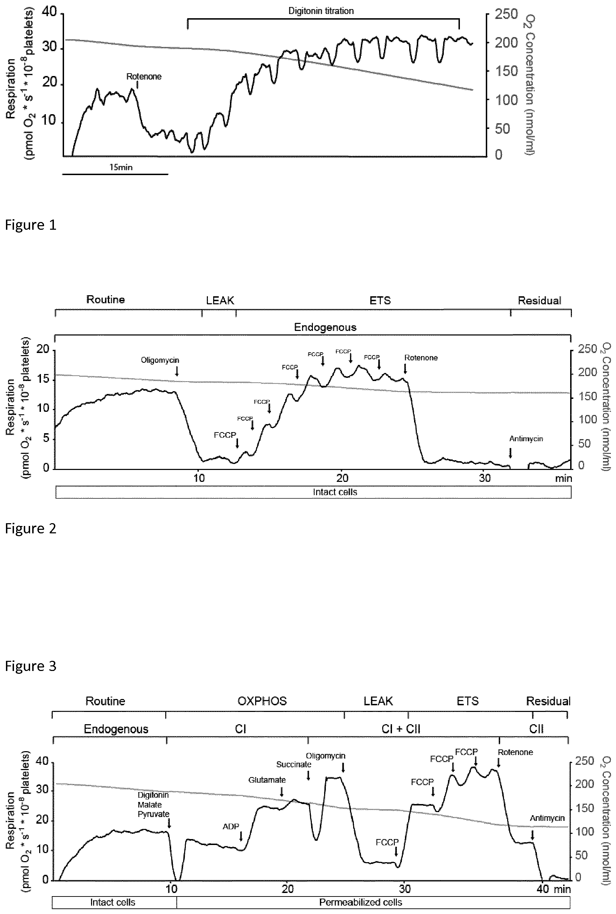

The complex I respiration (OXPHOS_{CI}) was stimulated by subsequent addition of ADP followed by additional complex I substrate glutamate (see Figure 3) followed by addition of increasing amounts of a test substance (e.g. metformin).

Complex II respiration was obtained by addition of a complex I inhibitor (e.g. rotenone) to a cell sample followed by addition of increasing amounts of a test substance.

Complex IV respiration was obtained by adding N,N,N',N'-tetramethyl-p-phenylendiamine (TMPD, 0.5 mM), an electron donor to complex IV. Due to the high level of autoxidation of TMPD, sodium azide (10 mM), an inhibitor of complex IV was added, and the difference between the two levels obtained was calculated as the specific complex IV activity.

A suitable method for investigating mitochondrial effects of drug candidates in clinical trials or in treatment regimens (i.e. items 3 and 4 above) is a method comprising
i) subjecting a sample of human blood cells containing mitochondria to high-resolution respirometry, wherein the sample of cells is from a person subjected to a clinical study or to a treatment regimen, and wherein a test substance has been administered to the person during the clinical study or treatment regimen
ii) adding to the cell sample a substance that increases the leakage of the inner mitochondrial membrane to protons,
iii) adding to the sample from ii) an inhibitor of mitochondrial complex I-function,
vi) adding to the sample from iii) an inhibitor of mitochondrial complex III-function, and
v) comparing the mitochondrial oxygen consumption of the sample from the person subjected to the clinical study or to a treatment regimen with the mitochondrial oxygen consumption of a control sample, wherein a decrease in mitochondrial oxygen consumption indicates a negative effect on the mitochondria, and wherein an increase in mitochondrial oxygen consumption indicates a positive effect on the mitochondria.

As seen from the above, the methods are almost identical and only vary with respect to which samples that are tested, i.e. whether it is a test substance or a cell sample of a person subject either to a clinical trial or to a treatment regimen. The person subjected to the clinical trial or to a treatment regimen receives a test or drug substance or a potential drug substance in accordance with the clinical trial or treatment regimen. This means that the mitochondria isolated from a blood sample from such a person have been exposed to the drug substance or potential drug substance in question and accordingly, any effect of such a substance on the mitochondrial respiration can be observed by the method described herein.

The mitochondrial respiration is followed by use of respirometry. Suitable settings are described in the examples herein, but a person skilled in the art will know how to change or adjust the settings if needed or if another apparatus is used.

The blood sample may be a venous blood sample. Platelets or white blood cells or a combination thereof may be isolated and used.

Suitable examples of substances that increase the permeability of the inner mitochondrial membranes to protons are carbonyl cyanide p-(trifluoromethoxy) phenylhydrazone (FCCP), carbonyl cyanide m-chlorophenylhydrazone (CCCP), 2,4-Dinitrophenol (DNP) or another protonophore.

In the examples herein and in the appended claims, further details are given for the methods.

In the following details with respect to the above methods are given.

### 1. Screening and selection of early or late stage drug candidates in cells derived from blood from healthy individuals or in so-called buffy coat, which is a concentrated solution of platelets and white blood cells

Such a method comprises
i) subjecting a sample of cells containing live mitochondria isolated from a human venous blood sample to high-resolution respirometry at an oxygen concentrations in the range of 400 - 25 µM O₂ at a constant temperature of 37°C
ii) contacting the sample of cells with a substance that increases the permeability of the inner mitochondrial membranes to protons such as carbonyl cyanide p-(trifluoromethoxy) phenylhydrazone (FCCP), carbonyl cyanide m-chlorophenylhydrazone (CCCP), 2,4-Dinitrophenol (DNP) or another protonophore to obtain maximal capacity of the electron transport system of the mitochondria present in the platelets,
iii) adding a test sample comprising a test substance in a vehicle; the addition is normally in stepwise increasing dosage, compared to vehicle addition,
iv) contacting the sample resulting from iii) with an inhibitor of mitochondrial complex I-function, such as rotenone, so as to elucidate the cellular respiration dependent on oxidation of complex II-substrates,
v) contacting the sample resulting from v) with the inhibitor of mitochondrial complex III-function, such as antimycin A, to determine any non-mitochondrial oxygen-consuming activity, such as auto-oxidation of said sample, and
iv) comparing the oxygen consumption before and after addition of the test sample and comparing the oxygen consumption between test and vehicle, wherein a decrease in oxygen consumption indicates a negative effect on the mitochondria.

The decrease may be significant or dose-dependent.

As mentioned above, the human blood sample is from healthy individuals. Normally the sample is obtained as described in the examples herein. The cells are normally either platelets of white blood cells or a combination thereof.

The cells may be suspended in human plasma (in some cases the subject's own plasma) or they may be suspended in aqueous buffer containing e.g. sucrose, HEPES, K-lactobionate, magnesium chloride, potassium dihydrogenphosphate, EGTA and BSA or other suitable buffers. pH is normally adjusted to pH between 7.0 and 7.5, notably 7.1. Normally, dissolution in plasma is preferred for intact platelets or for white blood cells in order to mimic the *in vivo* situation best possible. However, in certain situations, or as a complement to plasma-dissolution, a phosphate buffer saline with addition of 5 mM glucose (may be used for comparison with plasma runs), or a more intracellular type of buffer containing Kreb's cycle intermediates for permeabilized platelets or white blood cells can be used. The intracellular type would be used for permeabilized platelets (and WBCs) and where mitochondrial respiratory substrates would be present in excess (in saturable amounts) to maximize electron transport and increase the resolution of the assay.

The cells obtained from the human blood sample may be assayed either intact or permeabilized.

Different experimental protocols may be used dependent on whether intact or permeabilized platelets or white blood cells (SUIT protocol - Substrate-Uncoupler-Inhibitor Titration) are used. In general, intact platelets or white blood cells are used. Permeabilized platelets or white blood cells are used to gather as much information as possible of the capacity of the different respiratory complexes during one experiment. The permeabilization of the platelet is performed by subjecting the cells to digitonin or other substances that make the plasma membrane of the cells permeable to substrates and ADP. Other substances may be saponin or triton-x. As seen from the examples herein, an optimal dosage of digitonin was found to be 1 µg/ 1x 10⁶ platelets.

The cells are normally suspended in the glass chamber of the apparatus in a concentration of from 200 x 10⁶/ml to 400 x 10⁶/ml.

The test sample may be added several times in increasing concentrations in order to identify the minimal concentration that gives negative effect on the mitochondria (i.e. toxic effect). The initial and final concentration of the test substance depends on the potency of the substance, but will normally be in a range from the submicromolar to the millimolar.

Thus, the method can be used to determine which dose levels that are safe and which are not and, accordingly, should be avoided.

The method may also be used to compare two of more drug substances or candidate drug substances with each other in order to identify the substance with the safest profile. Thus, the method is repeated with the other substance(s) under investigation and the results obtained are compared. The substance having the lowest decrease in respiration compared to normal level is the safest substance regarding mitochondrial toxic effects.

Figures 7-9 show the result of such comparison. Figure 7 shows the results obtained from two experiments with antidiabetics; one involving titration with the drug substance troglitazone and the other experiment with the use of rosiglitazone. Figure 7 clearly shows that rosiglitazone is a safer drug substance than troglitazone with respect to mitochondrial toxicity. Troglitazone is no longer on the market due to toxic effects.

Figure 8 shows the results obtained from two experiments using minocycline and doxycycline, which indicate that doxycycline is safer than minocycline.

Figure 9 shows the results obtained from two experiments using cerivastatin and simvastatin, which show that simvastatin is safer than cerivastatin. In fact, cerivastatin has already been withdrawn from the market, whereas simvastatin is still on the market.

Carbonyl cyanide p-(trifluoromethoxy) phenylhydrazone (FCCP) or another substance that increases the permeability of the inner mitochondrial membrane to protons is added to obtain maximal capacity of the electron transport system of the mitochondria present in the platelets. As seen from the examples herein a concentration of from about 5 to about 100 µM gives suitable response. When plasma is used to dissolve the platelet pellet an optimal concentration was found to be about 100 µM with a suitable range of from about 50 to about 200 µM, and when phosphate buffered saline (PBS) was used a lower concentration was optimal, namely about 6 µM with a range of from 2 to about 20 µM.

At suitable point in time after the test substance has been added (such as from 1 to 10 min) a complex I (CI) inhibitor such as rotenone and a complex III (CIII) inhibitor such as antimycin-A are sequentially added to inhibit the electron transport system (ETS) providing the residual, non-mitochondrial, oxygen consumption, which is subtracted from the different respiratory parameters in further analyses. This procedure enables evaluation of specific mitochondrial respiration and may also reveal auto-oxidation properties of tested compounds.

Rotenone is added in an amount corresponding to a final concentration in the sample of about 2 µM with a range of from about 1 to about 5 µM. If another complex I inhibitor is used the final concentration should have a size leading to the same effect as if rotenone is used.

Antimycin-A is added in an amount corresponding to a final concentration in the sample of about 1 µg/ml with a range of from about 0.1 to about 10 µg/ml. If another complex III inhibitor is used the final concentration should have a size leading to the same effect as if Antimycin-A is used.

Antimycin-A is always added after rotenone in order to determine complex II activity following complex I inhibition.

The details of the experiments appear from the examples and claims herein.

### 2. Testing a patient's sensitivity to a known mitochondrial toxicant

Today many of the known mitochondrial toxicants are registered and used. However, the physician would like to know, before drug treatment is started, if the patient is sensitive to these drugs. One example is the widely-used anti-epileptic drug valproic acid, which can cause severe brain or liver damage if given to the wrong patients.

Such a method is in essence the same as described under item 1) above. The method comprises
i) subjecting cells containing live mitochondria isolated from a human venous blood sample to high-resolution respirometry at an oxygen concentrations in the range of 400 - 25 µM O₂ at a constant temperature of 37°C,
ii) contacting the cell sample with carbonyl cyanide p-(trifluoromethoxy) phenylhydrazone (FCCP) or another substance that increases the permeability of the inner mitochondrial membrane to protons to obtain maximal capacity of the electron transport system of the mitochondria present in the platelets,
iii) adding a test sample comprising said substance in stepwise increasing dosage, compared to vehicle addition,
iv) contacting the sample resulting from iv) with an inhibitor of mitochondrial complex I-function, such as rotenone, so as to elucidate the cellular respiration dependent on oxidation of complex II-substrates,
v) contacting the sample resulting from v) with the inhibitor of mitochondrial complex III-function, such as antimycin A, to determine any non-mitochondrial oxygen-consuming activity, such as auto-oxidation of said sample, and
vi) comparing the oxygen consumption before and after addition of the test sample, wherein a decrease in oxygen consumption indicates a negative effect on the mitochondria,

Here, the human blood sample is taken from the patient who may undergo a specific treatment with a specific drug substance or candidate drug substance and the test sample contains the specific drug substance or candidate drug substance.

As described above under 1), the test sample may be added several times in increasing concentrations in order to identify the minimal concentration that gives negative effect on the mitochondria (i.e. toxic effect). The initial and final concentration of the test substance depends on the potency of the substance, but will normally be up to 10 times the through or steady state levels to account for drug accumulation in tissues.

Thus, the method can be used to determine which dose levels that are safe and which are not and, accordingly, should be avoided.

The method may also be used to determine, which drug substance of a series of possibilities should be selected to the particular patient. Thus, the method may involve comparison of two of more drug substances or candidate drug substances with each other in order to identify the substance with the safest profile in the patient in question. Thus, the method is repeated with other substances and the results obtained are compared. The substance having the lowest decrease in respiration compared to normal level is the safest substance regarding mitochondrial toxic effects for the patient in question.

All the details relating to the individual parts of the method are as described herein above and reference is made thereto.

### 3. Analysing mitochondrial drug toxicity in clinical trials

The clinical trial can be any trial e.g. dose-finding studies, safety studies in humans etc. and the method is based on a simple blood sampling. Both short-term, acute, long-term, and chronic effects can be evaluated. A striking example is HIV medication, which can give symptoms of mitochondrial failure following long-term use (due to mitochondrial DNA-depletion). To this end it is a great advantage of the present method that is enables determination of combined toxicity of the drug substance as well as its circulating metabolites in the subject's plasma.

Such a method is essentially the same as described above, but the human blood sample is taken from the subject enrolled in the study and from a healthy individuals functioning as a control group. Accordingly, the subject has received a drug substance or a potential drug substance before the blood sample is taken. The mitochondrial respiration may be followed during the whole treatment regimen or clinical study by regularly taking a blood sample from the subject in question.

Thus, such a method more specifically comprises
i) subjecting cells containing live mitochondria isolated from a human venous blood sample to high-resolution respirometry at an oxygen concentrations in the range of 400 - 25 µM O₂ at a constant temperature of 37°C, wherein the human blood sample is obtained from a person subjected to a clinical study or to a treatment regimen, and wherein a test substance has been administered to the person during the clinical study or treatment regimen,
ii) contacting the cell sample with carbonyl cyanide p-(trifluoromethoxy) phenylhydrazone (FCCP) or another substance that increases the permeability of the inner mitochondrial membrane to protons to obtain maximal capacity of the electron transport system of the mitochondria present in the platelets,
iii) contacting the sample resulting from ii) with an inhibitor of mitochondrial complex I-function, such as rotenone, so as to elucidate the cellular respiration dependent on oxidation of complex II-substrates,
iv) contacting the sample resulting from iv) with the inhibitor of mitochondrial complex III-function, such as antimycin A, to determine any non-mitochondrial oxygen-consuming activity, such as auto-oxidation of said sample, and
v) comparing the oxygen consumption of the human blood sample from a person subjected to the clinical study or treatment regimen with the oxygen consumption of a human blood sample from a person from a control group, wherein a decrease in oxygen consumption compared with the control indicates a negative effect on the mitochondria.

In the present context a clinical study can be any study including pre-clinical studies and long-term safety studies. As seen from the above, the person may also be a patient who suffers from a disease or disorder and is subject to a treatment regimen with one or more drug substances or candidate drug substances.

The samples may be from one individual or it may be pooled samples from more individuals. In some cases the plasma is collected from patients undergoing treatment with a drug in clinical trials or otherwise. The plasma should be centrifuged and can be frozen and stored until analysis can be made. The plasma would contain parent drug as well as drug metabolites (which may be as toxic as or more to the mitochondria than the parent drug). The plasma would be thawed and healthy platelets or white blood cells (WBC) could be submerged in this plasma to see if it would affect mitochondrial function There are several benefits with this including that there is no need to transport blood from clinical trials urgently for analysis.

All the details relating to the individual parts of the method are as described herein and reference is made thereto.

### 4. Analysing beneficial effects of drugs intended to improve mitochondrial function

Large screening programs prior to clinical trials can be executed in cells derived from blood from healthy individuals or from buffy coat. Analysis can also be performed in the blood of the patient (often children) before treatment and during treatment and following changes in dosing.

The method is essentially identical to the method described in item 3, but the human blood samples are taken from the patients before and during treatment or after changes in dosing or administration route or form. The samples are analysed and the results compared. Thus, a first blood sample may be taken before treatment (or any change in the treatment) and a second blood sample may be taken during treament or after any change in the treatment. The effect of the treatment may also be follow by testing samples taken currently during the treatment.

Thus, such a method comprises
i) subjecting cells containing live mitochondria isolated from a human venous blood sample to high-resolution respirometry at an oxygen concentrations in the range of 400 - 25 µM O₂ at a constant temperature of 37°C, and wherein a test substance has been administered to the person before the blood sample is taken,
ii) contacting the cell sample with carbonyl cyanide p-(trifluoromethoxy) phenylhydrazone (FCCP) or another substance that increases the permeability of the inner mitochondrial membrane to protons to obtain maximal capacity of the electron transport system of the mitochondria present in the platelets,
iii) contacting the sample resulting from ii) with an inhibitor of mitochondrial complex I-function, such as Rotenone, so as to elucidate the cellular respiration dependent on oxidation of complex II-substrates,
iv) contacting the sample resulting from iv) with the inhibitor of mitochondrial complex III-function, such as antimycin A, to determine any non-mitochondrial oxygen-consuming activity, such as auto-oxidation of said sample,
v) comparing the oxygen consumption of a first human blood sample with the oxygen consumption of a second blood sample, wherein a decrease in oxygen consumption indicates a negative effect on the mitochondria and an increase in oxygen consumption indicates a positive effect on the mitochondria.

The result is compared with a control or reference.

Step i)-v) may be repeated with a third, fourth, fifth etc. sample dependent of the kind of change that is investigated.

The first sample may be a control sample, i.e. a blood sample taken before any treatment is initiated. Alternatively, the first and the second sample represent blood samples taken at different points in time during a treatment regimen. For example if a treatment regimen lasts a week, the first sample may be taken at day 1 (reference sample) and the second sample at day 2 (and further samples taken at the following days). If a dose regimen is changed, the first blood sample may be taken before the dose is changed and the second sample after the dose is changed.

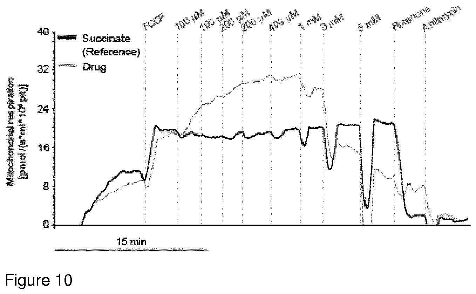

Figure 10 shows the experimental traces of beneficial and toxic effect on mitochondrial respiration of a potential drug candidate as compared to control.

All the details relating to the individual parts of the method are as described herein and reference is made thereto.

### Function of mitochondria

Mitochondrial dysfunction is recognized in primary respiratory chain diseases due to nuclear or mitochondrial DNA mutations and is also implicated in disorders such as Huntington's, Alzheimer's and Parkinson's disease as well as the result of excessive inflammation such as in sepsis (Brealey et al., 2002; Ferreira et al., 2010; Kones, 2010; Rosenstock et al., 2010).

The study of mitochondrial function is essential to both basic research of mitochondrial physiology and pathogenic mechanisms, as well as for the diagnostics of mitochondrial diseases. A common method to investigate mitochondrial function is determination of maximal enzymatic activity of the individual electron transport system (ETS) complexes in disrupted mitochondria by spectrophotometry. The benefit of this procedure is easy storage and transport to core laboratory facilities with high throughput analyses since the samples can be frozen (Haas et al., 2008). However, the mitochondrion and its components do not work as isolated units. Respiratory chain complexes are interconnected in the ETS that in turn gather to multi-enzyme- and super complexes (Lenaz and Genova, 2009). Mitochondria undergo fusion and fission, form networks and crosstalk with other subcellular compartments (Picard et al., 2011). This clearly highlights the need to analyse mitochondrial function without, or at least with minimal, cell disruption and with an as close as possible physiological environment. This can be achieved using polarographic measurements of mitochondrial respiration in cells (Kitchens and Newcomb, 1968). Respiration can be analysed in intact cells with natural surrounding media, such as plasma, utilizing endogenous substrates. Further, with permeabilization of the cell membrane direct access to the mitochondria for exogenous substrates and inhibitors can be achieved and individual complexes of the ETS investigated without the need for cell disruption and mitochondrial purification (Hutter et al., 2006).

When selecting which tissue to investigate for evidence of mitochondrial dysfunction the best choice is the tissue most profoundly affected by the disease process in a given patient. However, this is seldom possible due to the invasiveness and risks associated with biopsies from internal organs such as brain, liver and heart. Therefore other tissues are used and most commonly muscle cells and skin fibroblasts (Haas et al., 2008).

Platelets are an easily obtainable source of viable mitochondria and sampling is less invasive compared to muscle or skin biopsy. Platelet mitochondrial alterations have been demonstrated in a variety of diseases, primarily affecting other organ systems (Hauptmann et al., 2006; Kriqe et al., 1992) as well as in the process of ageing (Merlo Pich et al., 1996; Xu et al., 2007) and has therefore been proposed to serve as a potential marker of systemic mitochondrial dysfunction (Sjövall et al., 2010; Xu et al., 2007). Also, platelets are well suited for polarographic analysis (Kitchens and Newcomb, 1968; Sjövall et al., 2010).

### Development of a method according to the invention

The aim of the study reported in the experimental section was to establish a methodology and make an in depth assessment of normal platelet respiratory function *ex vivo* in intact viable cells and individual complex function in permeabilized cells using high-resolution respirometry. Secondly, the impact of storage in cold environment and room temperature were studied, influence of gender and age and thirdly, the consistency of the method applied to different reference cohorts.

For details relating to the experimental set-up reference is made to the experimental section.

Data are presented reflecting normal platelet mitochondrial respiratory function of several different reference cohorts. Oxidative phosphorylation accounts for approximately 85% of the energy production in the resting platelet (Kilkson et al., 1984) and besides glycolysis, β-oxidation of fatty acids also contributes to substrate supply (Cesar et al., 1987). Spare respiratory capacity of intact platelets, i.e. how much respiration can be increased by the uncoupler FCCP from the routine state, was higher in platelets suspended in plasma compared to PBS as indicated by the differences in ETS/routine (Table 2). This probably reflects the varying types and levels of substrate supply in the different media. The spare respiratory capacity of intact platelets was about 64%, when experiments were performed in PBS with glucose only as exogenous substrate, compared to approximately 88% when the cells were incubated and analysed in their own plasma where they have a physiologic substrate supply. When exposed to oligomycin, spare respiratory capacity was generally lower, about 28% in PBS-glucose and 25% in plasma, possibly reflecting compromised energy dependent oxidation pathways of glucose and other substrates in cells with inhibited ATP production.

In permeabilized cells, both glycolysis and β-oxidation are by-passed by providing saturating amounts of substrates to the citric acid cycle. The result is an ETS and proton circuit that can be evaluated without substrates being rate-limiting. Maximal oxygen consumption, either ADP- (coupled) or FCCP-stimulated (uncoupled), could be increased by an additional -50% in permeabilized cells compared to intact cells. This suggests a rate-limiting step of substrate supply in stimulated intact cells which is not present in the resting state since routine respiration was similar regardless of the suspending media. Intact platelets use NADH and electron input through complex I almost exclusively since no complex II driven respiration was detectable after complex I inhibition by rotenone. The finding that ETS respiration values of intact cells were in the same range as for OXPHOS_{CI} of permeabilized cells lend further support to this notion. This also emphasizes the importance of feeding the ETS with electrons from both complex I and complex II into the Q-junction in order to establish maximal electron transport in permeabilized cells. With unrestricted substrate supply, complex I contribute about 2/3 and complex II 1/3 of total respiration at convergent electron input. The ATP synthase was not observed to cause any significant rate limitation as maximal ADP- and FCCP-stimulated respiratory capacity were similar with an OXPHOS_{CI+II}/ETS_{CI+II} ratio close to one. Also, platelet respiration displayed a tight coupling to oxidative phosphorylation as demonstrated by low LEAK respiration in relation to maximal respiratory capacity and corresponding high ETS/LEAK ratios in both intact and permeabilized cells. We observed a higher LEAK state in permeabilized compared to intact cells. Although permeabilization of the plasma membrane with digitonin could affect the permeability of the mitochondrial membrane this is not supported by our findings as there was a large safety margin of digitonin concentration observed in the digitonin titration experiments. There was also no effect on respiration by exogenous cytochrome c and thus the mitochondrial outer membrane remained intact. A more likely explanation is that with saturating substrate supply a higher proton gradient across the inner membrane can be generated increasing LEAK respiration since this basal respiration rate stands in proportion to the proton-motive force (Nicholls, 1977).

Reduction in mitochondrial function with age has been widely implicated (Lesnefsky and Hoppel, 2006; Vendelbo and Nair, 2011). In platelets we observed a decrease in complex II respiration with age without an effect on the overall respiratory function, an effect mostly related to a difference between the paediatric and adult cohort. The decline in respiration seemed to be complex II specific since a more downstream effect on complex III, complex IV or the ATP-synthase also should have influenced maximal OXPHOS or ETS activity.

The difference between routine respiration and LEAK respiration is generally attributed to the resting ATP production. Since LEAK state remained at the same level throughout the age span studied, the increase in routine respiration observed herein would suggest an increased basal ATP production with age. Generally there is a decline in resting metabolic rate with age (Johannsen and Ravussin, 2010) but how it affects various tissues is not clearly elucidated. Why platelets would require an increased basal ATP production with age is not clear from the present study. Compared to the adult Swedish counterparts, the Japanese volunteers displayed an approximately 27% higher LEAK respiration. This resulted in a significantly increased ETS_{CII}/LEAK in complex II substrate driven respiration but a significantly reduced OXPHOS_{CI}/LEAK and OXPHOS_{CI+II}/LEAK. Japanese and Caucasians have been reported to display differences in disease patterns (Benfante, 1992). This has been explained by both genetic differences but also, due to food and lifestyle factors since the differences, to a large extent, disappear in a migrating population (Yamori, 2006). Japanese food is of tradition rich in fish and other seafood products that have a high content of FFA with uncoupling properties (Cha et al., 2001; Davis et al., 2008). Mild uncoupling has been suggested as beneficial in terms of ageing due to a reduced proton motive force and as a result less ROS production (Brand, 2000). Japanese mitochondrial DNA is dominated by haplogroup D compared to European where haplogroup H is predominant. With respect for the small sample size, the differences seen in our study could thus be either genetic or lifestyle related or a combination of both. Further studies are needed to clarify these matters. Respiration levels from umbilical cord samples were generally higher compared to the other cohorts. This is probably a reflection of the different requirements between intra- and extrauterine life. However, data on mitochondrial function in fetal life are scarce (Yanicostas et al., 2011).

The presented method could potentially serve as an adjunct to the present standard evaluation of suspected mitochondrial disease which usually involves muscle biopsies. As such we are currently collecting data from several different cohorts such as patients with neurodegenerative disorders and newborn children with suspected mitochondrial disorders. We therefore wanted to evaluate the limitations posed by storage and small sample volume. Even though storage in EDTA vials is not an optimized milieu for platelets, there seem to be only minor alterations in respiratory function after 24 h of storage in blood. For diagnostic purposes, a sample could thus potentially be transported to core facilities over longer distances with maintained possibility for analysis of respiratory capacity in viable platelets. In very small children there may be limitations to how much blood that can be drawn for different analyses and we therefore evaluated how low platelet concentrations we could use and still obtain reliable values. At 50 x 10⁶ platelets / ml the absolute oxygen consumption was only approximately 25 nmol / ml for a whole experiment. The respiratory states were not affected when evaluated at different concentrations with the exception of ETS_{CI + II}, which was slightly reduced at 50 x 10⁶ platelets / ml. The reason for this is not entirely clear but the maximal effect of FCCP and its impact of unspecific binding to other cell membranes may be altered with reduced cell content even if carefully titrated.

The strength of the present method is the ability to combine analysis of both intact and permeabilized platelets. Analyzing intact cells suspended in the subject's own plasma is an experimental setting likely very similar to physiological conditions. This makes it possible to study not only inherent genetic defects but also mitochondrial defects induced by exogenous factors such as toxins or pharmaceuticals. Further information can be gained from permeabilized cells where different titration (SUIT) protocols can be used to shift flux control to different parts of the respiratory system to elucidate where the pathology is situated.

Although respirometry can be considered as one of the preferred methods in assessing mitochondrial bioenergetic function (Brand and Nicholls, 2011), and platelet mitochondria seem to be a good source of viable human mitochondria, the method has limitations. Diseases affecting mitochondria may be more or less tissue specific and mutations in mtDNA are known to be expressed at different ratios in different tissues, a phenomenon known as heteroplasmy. As such, alterations could be unrecognized if not substantially present in the analysed tissue. Cells have the ability to compensate for decreasing levels of ATP and usually exhibit a threshold where compensation can no longer occur and organ failure ensues (Rossignol et al., 2003). It is thus difficult to predict at what level decreased mitochondrial respiration should be considered pathological. However, all the above limitations are not unique to the present method but exist regardless of what tissue or technique is being used. With the present titration protocol we did not include specific measurements of complex IV which is normally done by the artificial electron donor TMPD in conjunction with ascorbate to keep TMPD reduced. TMPD/ascorbate is subjected to auto oxidation catalyzed by different metal-containing proteins (such as cytochrome c) and is also oxygen concentration dependent. Due to the nature of the preparation of our samples, the amount of unspecific cell material and plasma varies and it is therefore not possible to make any background correction for TMPD /ascorbate auto-oxidation.

### Conclusion

Respiratory measurements of platelets are well suited for studying human mitochondria in *ex-vivo* physiologic conditions. It is demonstrated that reliable results can be obtained with minute sample amounts and after storage up to 24 h using high-resolution respirometry. By applying different SUIT protocols detailed information of the cells respiratory capacity and differences between different cohorts can be obtained and we describe how to evaluate these results. This approach may be suitable for evaluating exogenously triggered as well as endogenous mitochondrial disorders and we are currently evaluating the method for these purposes.

### Legends to figures

Fig 1. Representative trace of digitonin titration. Platelets at a concentration of 100 * 10⁶/ml suspended in MiR05 buffer initially containing succinate 5 mM and ADP 1 mM. After stabilisation at routine respiration complex I was inhibited by rotenone followed by stepwise (20 µg) digitonin titration until no further increase was detected.
Fig 2. Experimental protocol of intact platelets. The endogenous (routine) respiration was followed by induction of complex V-independent (LEAK) respiration by oligomycin (1 µg/ml). Maximal respiration was achieved by titration of the protonophore, carbonyl cyanide p-(trifluoromethoxy) phenylhydrazone (FCCP, in PBS mean concentration 6 µM, in plasma mean concentration 100 µM) followed by rotenone (2 µM) and subsequently antimycin-A (1 µg/ml), complex I and III inhibition, respectively, for measurement of residual oxygen consumption. Induced respiratory states and respiratory substrates used are indicated above the graph. ETS = electron transport system.
Fig 3. Experimental protocol of permeabilized platelets. Trace from experiment displaying oxygen consumption rate using a substrate, uncoupler, inhibitor titration protocol. Induced respiratory states and respiratory complexes activated are defined above the graph. Platelets were permeabilized with digitonin and the complex I (CI) substrates malate and pyruvate (5 mM, respectively) were simultaneously added. Oxidative phosphorylation (OXPHOS) was stimulated by subsequent addition of ADP (1 mM) followed by the additional complex I substrate glutamate (5 mM). Addition of the complex II (CII)-linked substrate succinate (10 mM) enabled convergent electron input via both complex I and complex II. OXPHOS was inhibited by oligomycin (1 µg/ml) revealing complex V-independent (LEAK) respiration. Maximal respiratory capacity of the electron transfer system (ETS) was induced by titration of the protonophore, carbonyl cyanide p-(trifluoromethoxy) phenylhydrazone (FCCP, mean concentration 6 µM)). Inhibition of complex I by rotenone (2 µM) revealed complex II-supported respiration. The residual non-mitochondrial oxygen consumption was exposed by addition of the complex III inhibitor antimycin-A (1 µg/ml). Addition of TMPD (0.5 mM) and azide (10 mM) is not displayed. Induced respiratory states and respiratory substrates used are indicated above the graph.

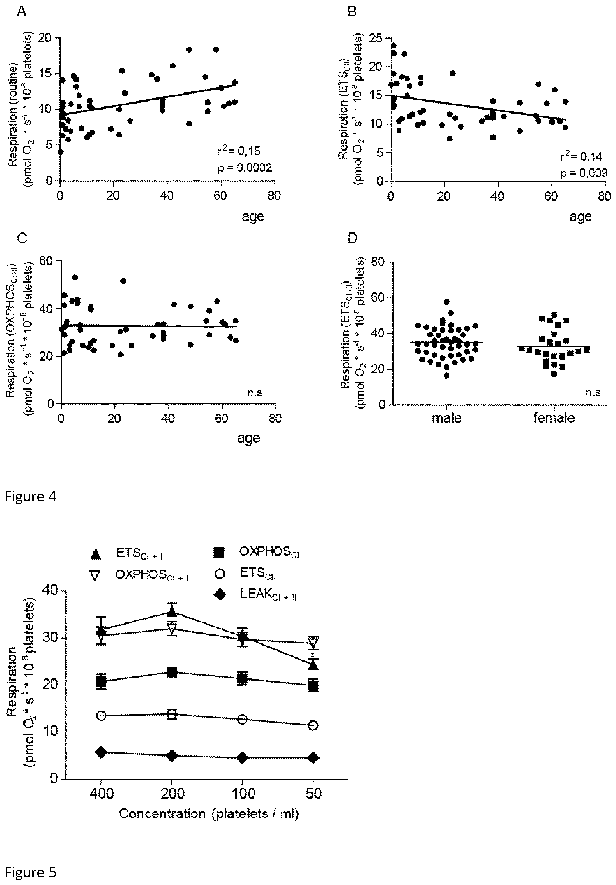

Fig 4. Correlation of age and sex with platelet mitochondrial respiration. A, correlation between routine respiration with age, individual points are means of duplicate values. B, correlation between ETSCII and C, OXPHOSCI+II respiration and age. D, Comparison of ETSCI+II respiration between gender. For definition of the respiratory states see Fig. 3.
Fig 5. Effect of platelet concentration on different respiratory states in permeabilized platelets. Oxygen consumption at the different states of the substrate, inhibitor titration protocol at concentrations ranging from 50 to 400 * 106 platelets / ml. For definition of the respiratory states see Fig. 3. N=5, each experiment was performed in duplicates, * = p < 0.05.

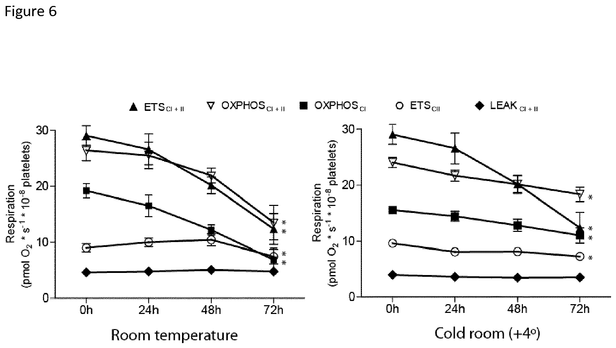

Fig 6. Effect of storage on different respiratory states in permeabilized platelets. Blood were stored in K2EDTA vacutainer tubes on tilting board in either room temperature or 4° C. At 72 h all parameters except LEAK respiration were significantly decreased compared to time 0. For definition of the respiratory states see Fig. 3. N=4, each experiment was made in duplicates, * = p < 0.05.

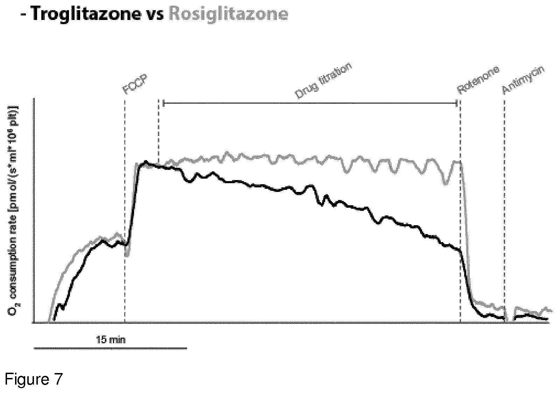

Fig 7. Experimental trace from mitochondrial toxicity assay of Troglitazone and Rosiglitazone. Human platelets at 200*10⁶ cells/ml in a buffer containing 110 mM sucrose, HEPES 20 mM, taurine 20 mM, K-lactobionate 60 mM, MgCl₂ 3 mM, KH₂PO₄ 10 mM, EGTA 0.5 mM, BSA 1 g/l, pH 7.1. Troglitazone displays mitochondrial toxicity as shown by decreased oxygen fluxes with higher concentrations. FCCP (2 µM), Rotenone (2 µM) and Antimycin (1µg/ml) added where indicated. FCCP = carbonyl cyanide p-(trifluoromethoxy) phenylhydrazone.

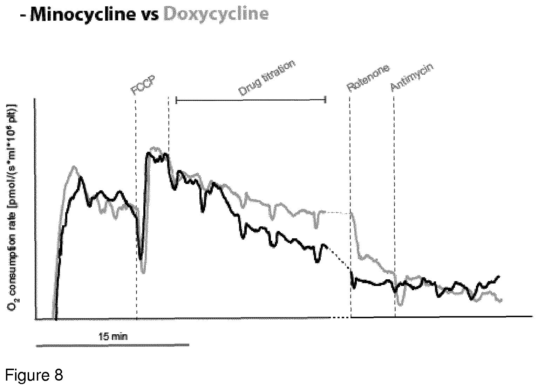

Fig 8 shows experimental trace from mitochondrial toxicity assay of Minocycline and Doxycycline. Human platelets at 200*10⁶ cells/ml in a buffer containing 110 mM sucrose, HEPES 20 mM, taurine 20 mM, K-lactobionate 60 mM, MgCl₂ 3 mM, KH₂PO₄ 10 mM, EGTA 0.5 mM, BSA 1 g/l, pH 7.1. Minocycline displays mitochondrial toxicity as shown by decreased oxygen fluxes with higher concentrations. Doxycycline also shows a toxicity but less pronounced as compared to Minocycline. FCCP (2 µM), Rotenone (2 µM) and Antimycin (1µg/ml) added where indicated. FCCP = carbonyl cyanide p-(trifluoromethoxy) phenylhydrazone.

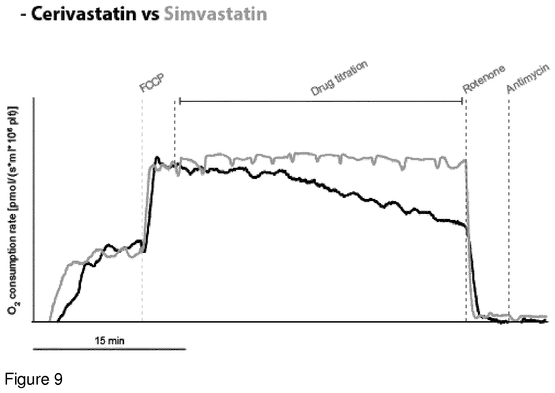

Fig 9 shows experimental trace from mitochondrial toxicity assay of Cerivastatin and Simvastatin. Human platelets at 200*10⁶ cells/ml in a buffer containing 110 mM sucrose, HEPES 20 mM, taurine 20 mM, K-lactobionate 60 mM, MgCl₂ 3 mM, KH₂PO₄ 10 mM, EGTA 0.5 mM, BSA 1 g/l, pH 7.1. Cerivastatin displays mitochondrial toxicity as shown by decreased oxygen fluxes with higher concentrations. FCCP (2 µM), rotenone (2 µM) and antimycin (1µg/ml) added where indicated. FCCP = carbonyl cyanide p-(trifluoromethoxy) phenylhydrazone.
Fig. 10 shows experimental traces of beneficial and toxic effects on mitochondrial respiration of a novel drug candidate as compared to control. Human platelets at 200*10⁶ cells/ml in a buffer containing 110 mM sucrose, HEPES 20 mM, taurine 20 mM, K-lactobionate 60 mM, MgCl₂ 3 mM, KH₂PO₄ 10 mM, EGTA 0.5 mM, BSA 1 g/l, pH 7.1. FCCP (2 µM), rotenone (2 µM) and antimycin (1µg/ml) added where indicated. Dose added at each titration step is denoted above the trace. The proposed drug increases mitochondrial respiration to 2mM accumulated dose whereafter the respiration decreases with further titration, indicating mitochondrial toxicity. FCCP = carbonyl cyanide p-(trifluoromethoxy) phenylhydrazone
Fig. 11 is a schematic figure of mitochondrial complex II screening assay. It shows the protocol for evaluating novel cell-permeable mitochondrial substrates. In the assay, mitochondrial function in intact cells is repressed with the respiratory complex I inhibitor rotenone. Drug candidates are compared with endogenous substrates before and after permeabilization of the plasma membrane to evaluate bioenergetic enhancement or inhibition.

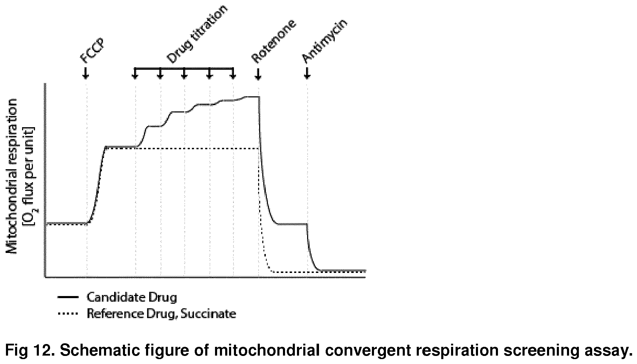

Fig. 12 is a schematic figure of mitochondrial convergent respiration screening assay. It describes the protocol for evaluating the potency of novel cell-permeable mitochondrial substrates. In the assay, mitochondrial activity is stimulated by uncoupling the mitochondria with the protonophore FCCP. Drug candidates are titrated to obtain the level of maximum convergent (complex I- and complex II-derived) respiration. After rotenone addition, complex II-dependent stimulation is obtained. The complex III-inhibitor antimycin is added to evaluate non mitochondrial oxygen consumption.

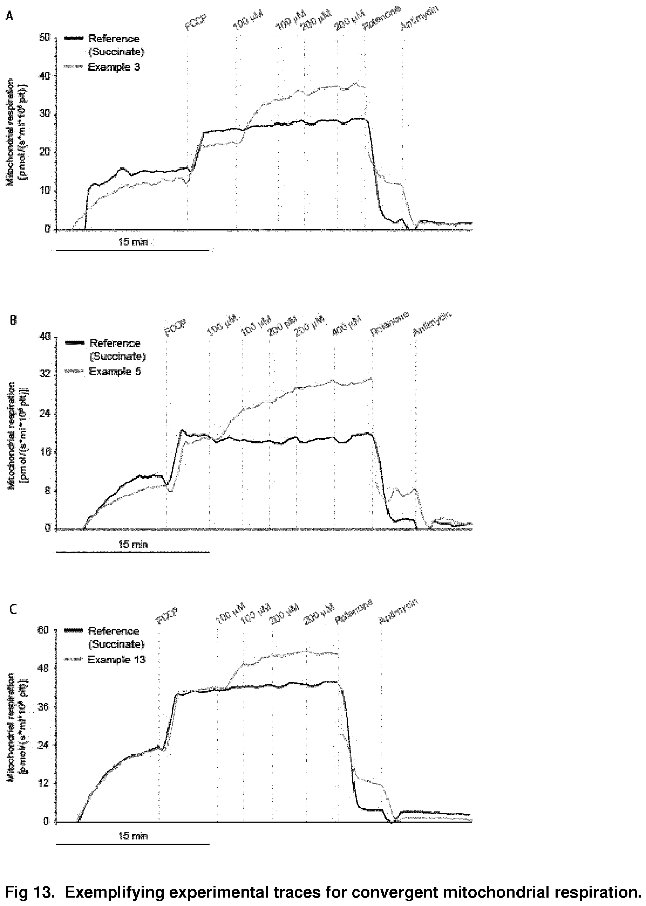

Fig. 13 shows the increase in respiration (oxygen flux per unit) with stepwise titration of drugs compared to control (disodium succinate) in intact human platelets (assay described in Fig 12).
Fig. 14 shows oxygen consumption dependent on mitochondrial complexes I, II and IV, respectively. The oxygen consumption was measured at different concentrations of metformin present in buffer with methods as described above. With increasing concentrations of Metformin, a significant decrease in Complex I respiration is seen. Data are expressed as mean and standard deviation. * p < 0.05, ***: p < 0.001 compared to vehicle (0 mM Metformin). Human platelets at 200*10⁶ cells/ml in a buffer containing 110 mM sucrose, HEPES 20 mM, taurine 20 mM, K-lactobionate 60 mM, MgCl₂ 3 mM, KH₂PO₄ 10 mM, EGTA 0.5 mM, and BSA 1 g/l, pH 7.1.

### Experimental section

### Materials and methods

### 1.1 Human sample acquisition.

The study was approved by the regional ethical review board of Lund, Sweden (adults: 113/2008, and 644/2009, children: 59/2009) and the ethics committee of Tokyo Medical University, Japan (permit no. 1514). For Swedish adults, blood samples were collected from healthy blood donors at the blood donor central, Skane University Hospital, Lund and healthy adults undergoing rehabilitation after knee injury. The Japanese cohort consisted of healthy adult volunteers. Samples were obtained after written informed consent was acquired. The experiments were carried out according to the same procedures and protocols and by the same researchers at both investigation sites. The paediatric control samples were obtained from patients undergoing minor elective surgery. Written informed consent was acquired from parents or guardian and blood was drawn before induction of anaesthesia. Umbilical cord blood was sampled after delivery from healthy individuals undergoing a normal pregnancy. Samples were obtained after written informed consent was acquired.

All chemicals were purchased from Sigma-Aldrich (St Louis, MO, USA) if not stated otherwise.

### 1.2 Platelet preparation

For blood donors, samples were taken from the collection tubing at the same time as a planned blood donation and in the other adult cohorts and children via venous puncture. Umbilical cord blood was sampled directly after the child was delivered either vaginally or by caesarean section. A volume of 21 ml, from adults, 6-12 ml, from children, and 3-6 ml from umbilical cord was drawn in K₂EDTA tubes (Vacuette®, Greiner Bio-One GmbH, Kremmünster, Austria). In pilot studies, K₂EDTA were shown to result in the best yield and prohibit platelet activation compared to Heparin, Citrate and Acid Citrate Dextrose (ACD) as anticoagulants (data not shown). Blood samples were freshly prepared and analyzed within 3-5 h. The tubes were centrifuged 15 min at 300 x g in room temperature, to yield a platelet-rich plasma (PRP). This PRP was pipetted off and centrifuged for 5 min at 4600 x g, at room temperature, producing a close to cell free plasma and a platelet pellet. The pellet was dissolved in 1-3 ml of the control subject's own plasma by gentle pipeting to obtain a highly enriched PRP with a mean final concentration of 1864 x 10⁶/ml (range 941 - 2498).

### 1.3 High-resolution respirometry

Respiration was measured at a constant temperature of 37°C in a high-resolution oxygraph (Oxygraph-2k Oroboros Instruments, Innsbruck, Austria (Gnaiger et al., 2000)) in 2 ml glass chambers with stirrer speed 750 rpm. Data was recorded with DatLab software 4.3. (Oroboros Instruments, Innsbruck, Austria) with sampling rate set to 2 s. All experiments were performed at an oxygen concentration in the range of 210 - 50 µM O₂. If necessary, reoxygenation was performed by partially raising the chamber stopper for a brief air equilibration. Instrumental background oxygen flux was measured in a separate set of experiments and automatically corrected for in the ensuing experiments according to the manufacturer's instructions. For respiration measurements in permeabilized cells, platelets were suspended in a mitochondrial respiration medium (MiR05) containing sucrose 110 mM, HEPES 20 mM, taurine 20 mM, K-lactobionate 60 mM, MgCl₂ 3 mM, KH₂PO₄ 10 mM, EGTA 0.5 mM, BSA 1 g/l, pH 7.1 (Gnaiger et al., 2000). For experiments in intact cells, platelets were suspended in either phosphate buffered saline (PBS) with addition of 5 mM glucose or in the control subject's own plasma. Calibration at air saturation was performed each day before starting experiments by letting Millipore water or respiration media stir with air in the oxygraph chamber until equilibration and a stable signal was obtained. Oxygen concentration was automatically calculated from barometric pressure and solubility factors that were set to 1.0 for water, 0.92 for MIR05 and PBS glucose and 0.89 for plasma (Baumgärtl and Lübbers, 1983).

### 1.3.1 Experimental protocol for intact platelets

Integrated respiration of intact cells with endogenous mitochondrial substrates was evaluated with two different titration protocols. Platelets were suspended in either PBS-glucose or the control subject's own plasma. Initially, samples were left to stabilise at a routine respiration state, revealing resting cellular energy demands on oxidative phosphorylation (OXPHOS). To evaluate the contribution of respiration independent of ADP phosphorylation, oligomycin (1 µg/ml, ATP-synthase inhibitor) was sequentially added inducing LEAK respiration state (also known as oligomycin-induced state 4 respiration). Maximal capacity of the ETS was measured after careful titration of the protonophore, carbonyl cyanide p-(trifluoromethoxy) phenylhydrazone (FCCP) until no further increase in respiration was detected (in PBS mean concentration 6 µM, in plasma mean concentration 100 µM). Rotenone (2 µM, complex I [CI] inhibitor) and antimycin-A (1 µg/ml, complex III [CIII] inhibitor) were then sequentially added to inhibit the ETS providing the residual oxygen consumption which was subtracted from the different respiratory parameters in further analyses.

In order to evaluate the influence on maximal respiratory capacity by the inhibition of ATP-synthase, a second experimental protocol was performed, where ETS capacity was evaluated by direct titration of FCCP after stabilization of routine respiration, followed by the same inhibitors as above. Control ratios were derived from maximal, FCCP-stimulated, respiration divided by LEAK respiration (ETS/LEAK) and routine respiration (ETS/routine).

### 1.3.2 Experimental protocol for permeabilized platelets

To access the ETS with saturating exogenous substrates and inhibitors the plasma membrane was permeabilized with the detergent digitonin. A set of experiments were performed to establish the optimal concentration of digitonin to induce maximal permeabilization of the plasma membrane without affecting the outer or inner mitochondrial membrane. Platelets (200 * 10⁶ / ml) were suspended in MIR05 and preincubated with ADP 1 mM, succinate 5 mM and rotenone 1 µM. Digitonin 10 µg/µl was titrated until the maximal response in respiration was obtained (Gnaiger et al., 1998). A representative graph of a titration experiment is shown in Fig. 1. The optimal dosage was found to be 1 µg / 1 x 10⁶ platelets. Exogenous cytochrome c did not induce any significant effect on respiration indicating that the mitochondrial outer membrane had remained intact (data not shown).

A substrate, uncoupler, inhibitor titration (SUIT) protocol was used to establish the respiratory capacities with electron flow through both complex I and complex II (CII) separately as well as convergent electron input via the Q-junction (CI + II) (Gnaiger, 2009). After routine respiration was established, titration was started with permeabilization of the plasma membrane with digitonin and a concomitant addition of malate (5 mM) and pyruvate (5 mM). OXPHOS capacity of complex I, driven by NADH-related substrates, was evaluated by adding ADP (1 mM), and additionally glutamate (5 mM) (OXPHOS_{CI}, or state 3_{CI}). Sequentially, 10 mM succinate was added inducing maximal OXPHOS capacity with convergent input through both complex I and complex II (OXPHOS_{CI + II}, or state 3_{CI+II}). Oligomycin (1 µg/ml) was used to inhibit the ATP synthase and induce LEAK respiration. Maximal convergent respiratory capacity of the ETS was subsequently obtained by titrating FCCP (ETS_{CI + II}, mean concentration 6 µM). Complex I was inhibited by rotenone (2 µM) to assess the ETS capacity supported by succinate through complex II only (ETS_{CII}). Finally, electron flow through the ETS was inhibited by addition of antimycin-A (1 µg/ml) providing the residual oxygen consumption not related to the ETS. Control ratios were derived from maximal oxidative respiration or maximal FCCP-stimulated respiration divided by LEAK respiration (OXPHOS_{CI + II} / LEAK and ETS_{CI + II}/LEAK respectively). Analysed samples were stored at -80 °C.

### 1.4 Data analysis

Statistical evaluation was performed using Graph Pad PRISM (GraphPad Software version 5.01, La Jolla, CA, USA). Values are presented as mean ± SEM, or individual values. All values from the different cohorts, except for umbilical cord and control ratios for intact cells (ETS/LEAK, ETS/routine), were found to be normally distributed with D'Agostino and Pearson omnibus normality test. Comparison between multiple groups was performed by one-way ANOVA with post hoc analysis between groups by Tukey's Multiple Comparison test, for parametric data, and Kruskal-Wallis test with Dunn's multiple comparison test for non-parametric data. For correlation with age, linear regression was used. A p-value < 0.05 was considered statistically significant.

### 2. Results

Blood samples were taken from 46 healthy adult volunteers (24 from Sweden and 22 from Japan), 28 male and 18 female, with median age 37 years (range 19-65 years) and 25 children, 18 male and 7 female, with median age 4 (range 1 month-12 years) and 22 umbilical cords (13 from caesarean section and 9 from vaginal delivery).

### 2.1 Yield, viability and intactness of platelet mitochondria

Platelet concentration was measured from whole blood and the yield calculated after preparation of the highly enriched platelet-rich plasma, was on average 92% (± 8%, n = 16). In order to evaluate viability of platelets after the preparation protocol, respirometry was performed on platelets centrifuged one step, i.e. 300 x g for 15 min and compared to respiration after pelletation. No significant differences could be seen concluding good stability and viability of the platelets throughout the isolation process (N = 16 data not shown).

### 2.2 Mitochondrial respiration of intact platelets

A representative graph of an experiment in intact cells is shown in Fig. 2 and values of respiratory parameters are displayed in Table 1. In intact cells the respiration is only driven by endogenous substrates. Routine respiration was similar in cells incubated in either PBS-glucose or plasma. LEAK respiration was very low, below 1 pmol O₂ /s /10⁸ platelets. The consequently high control ratio ETS/LEAK indicates a very tightly coupled respiration. Maximal stimulation of the ETS with FCCP was significantly higher in experiments without oligomycin and spare respiratory capacity, as indicated by the control ratio ETS/Routine, was significantly higher in non-oligomycin treated platelets suspended in plasma compared to PBS glucose. Inhibition of complex I with rotenone inhibited respiration by ∼ 99% and no further inhibition was seen after the addition of antimycin-A (Table 1). Between the different cohorts there was a significant difference between maximal FCCP-stimulated respiration (ETS) in Japanese controls versus umbilical cord blood (15 ± 1.3 vs 23 ± 2.3 pmol O₂ / s / 1 * 10⁶ platelet). No other significant differences were seen.

### 2.2.1 Mitochondrial respiration of permeabilized platelets

The SUIT protocol was developed to acquire as much information possible of the capacity of the different respiratory complexes from a single experiment. A representative trace with substrate and inhibitor additions and definitions of the different states is depicted in Fig. 3. Routine respiration of cells in MiR05 was in the same range as for intact platelets incubated in PBS-glucose or plasma. After addition of digitonin a steady decline of respiration was seen as cytosolic substrates and adenine nucleotides diffused into surrounding media. In the presence of malate and pyruvate as complex I substrates, ADP stimulation increased respiration by ∼ 80% compared to routine respiration. With glutamate added for additional NADH generation and complex I electron input, respiration increased by another - 10% (Table 2). After addition of succinate, convergent electron flow with input from both complex I and complex II to the ETS was achieved, and resulted in respiration rates three times that of routine respiration and a ∼ 50% increase from respiration with only complex I substrates. LEAK respiration was ∼ 15% of maximal coupled respiration, OXPHOS_{CI+II}. The ratio OXPHOS_{CI+II}/LEAK of -7.0 indicated good coupling of electron transport to ATP synthesis and is comparable to other tissues (Kuznetsov et al., 2002) (Table 2). Maximal respiratory capacity, ETS_{CI+ II}, induced by the protonophore FCCP, was - 5 % higher compared to OXPHOS_{CI+II}. The OXPHOS/ETS ratio was close to one and indicates that almost no flux limitation is exerted by the phosphorylation system at saturating exogenous substrates. ETS_{CII} activity, measured after inhibition of complex I by rotenone, was - 35% of the combined activities of complex I and complex II, ETS_{CI+II}. In the Japanese reference material, maximal respiration with complex I substrates as well as convergent input was similar to their Swedish counterparts. However, both LEAK respiration and complex II stimulated respiration were ∼ 25% higher compared to the values from the Swedish and paediatric cohorts resulting in a decreased ETS CI+II/LEAK ratio (Table 2). The values from umbilical cord blood differed by exhibiting higher values of maximal respiration at both OXPHOS as well as ETS. LEAK respiration was also significantly higher both in absolute values as well as proportionally, since the resulting ratios were lower (Table 2).

### 2.3 Age and sex correlation

Two respiratory states demonstrated significant correlations with age. Routine respiration increased (r² = 0.15, p<0.05) and ETS_{CII} decreased slightly (r² = 0.14, p<0.05) with age (umbilical cord data not included) as shown in Fig. 4A, B. Between all other respiratory parameters, there were no significant changes, exemplified with OXPHOS_{CI+II} in Fig. 4C. No correlation between respiratory parameters and gender were seen neither in intact nor permeabilized platelets (Fig. 4 D).

### 2.4 Linearity of respiration at different platelet concentrations

Respiration at different platelet concentrations were evaluated. In the range of 100 - 400 x 10⁶ pit /ml respiration remained linear in all states. At a concentration of 50 x 10⁶ pit /ml maximal FCCP stimulation was decreased by 20-25% (Fig. 5). The majority of further experiments was performed with a platelet concentration of 200 x 10⁶ /ml. The method showed good reproducibility when evaluated by experiments performed from the same individuals at separate days. The coefficient of variation was 6-13% in the different respiratory parameters (Table 3).

### 2.5 The influence of prolonged blood storage on respiratory parameters

The effect of whole blood storage on mitochondrial respiration was evaluated. Freshly drawn blood was stored in EDTA vials in either room temperature or at 4°C on a tilting board for up to 72 h. After 24 h respiration remained stable. At 48 h there was a clear trend towards reduced respiratory capacity of the substrate stimulated respiration states i.e. OXPHOS_{CI}, OXPHOS_{CI + II} and ETS_{CI + II}. At 72 h all parameters except LEAK respiration had declined significantly (Fig. 6).

### 3. Protocol for white blood cells (WBC)

### 3.1 Sample preparation

In patients, a maximal volume of 40 mL of blood was drawn from an existing arterial line in K₂EDTA tubes (Vacuette®, Greiner Bio-One GmbH, Kremmünster, Austria). In controls, blood samples were taken via venous puncture in K₂EDTA tubes. Leukocytes were isolated from whole blood by Ficoll gradient (Böyum REF) centrifugation. After washing in normal saline, cells were resuspended in 200 - 400 µl of saline, depending on yield, together with 50 - 100 µl of the subject's own plasma. Respirometric measurements were performed within 5 hours of sampling. The analyzed contents from the respirometry chamber were stored frozen until further use.

### 3.2 High-resolution respirometry

Leukocytes were placed in the 2 ml oxygraph chamber at a final concentration of 2.5 - 5 * 10⁶ cells/ml. In intact cells respiration media consisted of the subject's own plasma and for permeabilized cells a respiration media containing sucrose 110 mM, HEPES 20 mM, taurine 20 mM, K-lactobionate 60 mM, MgCl₂ 3 mM, KH₂PO₄ 10 mM, EGTA 0.5 mM, BSA 1 g/l, pH 7.1 (MiR05) [Gnaiger et al., 20001. Measurements were performed at a constant temperature of 37°C in a high-resolution oxygraph (Oxygraph-2k Oroboros Instruments, Innsbruck, Austria). Oxygen concentration (µM) and oxygen flux (negative time derivative of oxygen concentration; pmol O₂ * S⁻¹ * 10⁻⁶ cells) was recorded with DatLab software 4.3. (Oroboros Instruments, Innsbruck, Austria). All experiments were performed at an oxygen concentration in the range of 210 - 50 µM O₂. Calibration at air saturation was performed each day. Instrumental background oxygen flux was measured in a separate set of experiments and automatically corrected for in the ensuing experiments according to the manufacturer's instructions.

Oxygen concentration was automatically calculated from barometric pressure and solubility factors that were set to 1.0 for water, 0.92 for MIR05 and 0.89 for plasma.

Three substrate-uncoupler-inhibitor-titration (SUIT) protocols were used, one in intact cells and two in permeabilized cells [Pesta and Gnaiger, 2012] In intact cells stimulation with exogenous substrates is limited due to poor permeability over the plasma membrane. Respiration is therefore maintained by endogenous substrates only. Cells were suspended in the subject's own plasma and were left for routine respiration to stabilize. Oligomycin (1 µg/ml) were added to the respiration chamber to induce a state 4 like respiration (LEAK or State 4u) were respiration is primarily due to leakage of protons over the inner mitochondrial membrane. Maximal oxygen flux was subsequently obtained by stepwise (20-40 µM) titration of the uncoupler FCCP. Finally complex I and complex III were inhibited by adding rotenone (2 µM) and subsequently antimycin-A (1 µg/ml). The residual oxygen flux was subtracted from mitochondrial respiration steady state values. The second and third SUIT protocol were performed in permeabilized cells. The initial steps were the same for both protocols. After cells had stabilized at routine respiration, the plasma cell membrane was permeabilized with the addition of digitonin (3 µg/1*10⁶ cells, optimum concentration evaluated in a different set of experiments, data not shown). Simultaneously, complex I substrates malate and pyruvate (5 mM respectively) were added to the respiration chamber. The ensuing addition of ADP (1 mM) stimulated respiration and represents oxidative phosphorylation capacity (OXPHOS or State 3) for that specific, NADH-linked, substrate combination. Pyruvate is converted to acetyl-CoA by pyruvate dehydrogenase in the mitochondrial matrix and hence OXPHOS capacity could be restricted with a defective working or inhibited enzyme. With the subsequent addition of glutamate (5 mM), pyruvate dehydrogenase is/was bypassed and maximal NADH-linked complex I respiration, OXPHOS_{CI}, was obtained. The subsequent addition of succinate (10 mM) stimulated OXPHOS_{CI+II} with convergent input of electrons through both complex I and complex II via the Q-junction. From here the SUIT protocols diverged. In SUIT-2 complex I-linked respiration was inhibited by rotenone (2 µM) rending OXPHOS_{CII} capacity with electron input through complex II only. Subsequently the electron transport system (ETS) was inhibited at complex III by adding antimycin-A and the residual oxygen flux was subtracted from mitochondrial respiration steady-states.

In SUIT-3, following maximal OXPHOS_{CI+II} capacity, ATP-synthase was inhibited by oligomycin (1 µg/ml) in order to evaluate LEAK_{CI+II} (state 4) respiration which is predominantly caused by proton slip or leakage over the inner mitochondrial membrane. Maximal electron transportation through respiration without oxidative phosphorylation, ETS_{CI+II}, was evaluated by stepwise (2-4 µM) titration of FCCP. Complex II-linked respiration, not coupled to oxidative phosphorylation, ETS_{CII}, was evaluated by adding rotenone and subsequently ETS was inhibited by antimycin-A. At this point reoxygenation was performed, in both SUIT-2 and -3 protocols to a level of 160-180µM O₂. The activity of complex IV was evaluated by adding N,N,N',N'-tertamethyl-p-phenyldiamine (TMPD 0.5 mM), an electron donor to complex IV. Due to auto oxidation of TMPD sodium azide (10 mM), an inhibitor of complex IV was added and the difference between the two levels obtained was calculated as the complex IV activity.

### In the following more specific protocols for screening for potential drug candidates are given

Two screening protocols were utilized.
(1) Initial screen, complex II-effect, was performed with isolated platelets or white blood cells in a buffer containing 110 mM sucrose, HEPES 20 mM, taurine 20 mM, K-lactobionate 60 mM, MgCl₂ 3 mM, KH₂PO₄ 10 mM, EGTA 0.5 mM, BSA 1 g/l, pH 7.1. After baseline respiration with endogenous substrates was established, complex I was inhibited with Rotenone 2 mM. Drug candidates dissolved in DMSO were titrated in steps to 100 µM, 500 µM and 5 mM final concentration. Subsequently, cell membranes were permeabilized with digitonin (1mg/1*10⁶ pit). After stabilized respiration, Succinate 10mM was added and after the respiration stabilized the experiment was terminated by addition of antimycin at final concentration 1 µg/mL and the residual respiration measured. Figure 11 shows the protocol for evaluating novel cell-permeable mitochondrial substrates. In the assay, mitochondrial function in intact cells is repressed with the respiratory complex I inhibitor rotenone. Drug candidates are compared with endogenous substrates before and after permeabilization of the plasma membrane to evaluate bioenergetic enhancement or inhibition. Figure 11 is a schematic figure of mitochondrial complex II screening assay. It shows the protocol for evaluating novel cell-permeable mitochondrial substrates. In the assay, mitochondrial function in intact cells is repressed with the respiratory complex I inhibitor rotenone. Drug candidates are compared with endogenous substrates before and after permeabilization of the plasma membrane to evaluate bioenergetic enhancement or inhibition.
(2) In the second protocol, convergent respiration, the same respiration buffer and cell concentrations as described above was used. After basal respiration was established, the mitochondrial uncoupler FCCP was added at a concentration of 2 mM. Drug candidates dissolved in DMSO were titrated in steps to 100 µM, 200 µM, 400 µM, 600 µM, 1 mM, 2 mM, 5 mM and 10 mM final concentration. The concentration needed to reach maximum convergent respiration was noted. The experiment was terminated by addition of 2 µM rotenone and 1 µg/mL antimycin and residual respiration measured. Figure 12 describes the protocol for evaluating the potency of novel cell-permeable mitochondrial substrates. In the assay, mitochondrial activity is stimulated by uncoupling the mitochondria with the protonophore FCCP. Drug candidates are titrated to obtain the level of maximum convergent (complex I- and complex II-derived) respiration. After rotenone addition, complex II-dependent stimulation is obtained. The complex III-inhibitor antimycin is added to evaluate non mitochondrial oxygen consumption. Figure 12 is a schematic figure of mitochondrial convergent respiration screening assay. It describes the protocol for evaluating the potency of novel cell-permeable mitochondrial substrates. In the assay, mitochondrial activity is stimulated by uncoupling the mitochondria with the protonophore FCCP. Drug candidates are titrated to obtain the level of maximum convergent (complex I- and complex II-derived) respiration. After rotenone addition, complex II-dependent stimulation is obtained. The complex III-inhibitor antimycin is added to evaluate non mitochondrial oxygen consumption.

In the convergent respiration screening assay an ideal compound displays a higher respiration with stepwise titration compared to control in uncoupled mitochondria in intact cells. Please refer to schematic protocol in Fig 12 and exemplifying graphs from experiments with example compounds number 3, 5 and 13 in Fig 13. Figure 13 shows the Increase in respiration (oxygen flux per unit) with stepwise titration of drugs compared to control (disodium succinate) in intact human platelets (Fig 12.).

### Properties of desired compound

(1) The ideal compound stimulates respiration in rotenone-inhibited intact at low concentration in the CII-screening protocol without inhibitory effect on succinate stimulated respiration after permeabilization. After inhibition of respiration with mitochondrial toxins, respiration should be halted. Please refer to Fig 1. and the listing below.
a > b means that a is greater than b
a >> b means that a is much greater than b
a → b means that the value of a is approaching the value of b

Desired properties of compounds:
- maximum value of a reached at low drug concentration.
- a >> a'
- a → b'
- c → c'
- d → d'

Compounds impermeable to the cellular membrane are identified in the assay as:
- a → a'

Non mitochondrial oxygen consumption induced by drug candidate is identified when
- d > d'

### References

Baumgärtl, H., Lübbers, D., 1983. Microaxial needle sensor for polarographic measurement of local O2 pressure in the cellular range of living tissue. Its construction and properties. , in: Gnaiger, E., Forstner, H. (Eds.), Polarographic Oxygen Sensors. Springer, Berlin, Heidelberg, New York, pp. 37-65.
Benfante, R., 1992. Studies of cardiovascular disease and cause-specific mortality trends in Japanese-American men living in Hawaii and risk factor comparisons with other Japanese populations in the Pacific region: a review. Human biology 64, 791-805.
Boushel, R., Gnaiger, E., Schjerling, P., Skovbro, M., Kraunsoe, R., Dela, F., 2007. Patients with type 2 diabetes have normal mitochondrial function in skeletal muscle. Diabetologia 50, 790-796.
Brand, M.D., 2000. Uncoupling to survive? The role of mitochondrial inefficiency in ageing. Experimental gerontology 35, 811-820.
Brand, M.D., Nicholls, D.G., 2011. Assessing mitochondrial dysfunction in cells. Biochem. J. 435, 297-312.
Brealey, D., Brand, M., Hargreaves, I., Heales, S., Land, J., Smolenski, R., Davies, N.A., Cooper, C.E., Singer, M., 2002. Association between mitochondrial dysfunction and severity and outcome of septic shock. Lancet 360, 219-223.
Cesar, J., DiMinno, G., Alam, I., Silver, M., Murphy, S., 1987. Plasma free fatty acid metabolism during storage of platelet concentrates for transfusion. Transfusion 27, 434-437.
Cha, S.H., Fukushima, A., Sakuma, K., Kagawa, Y., 2001. Chronic docosahexaenoic acid intake enhances expression of the gene for uncoupling protein 3 and affects pleiotropic mRNA levels in skeletal muscle of aged C57BL/6NJcI mice. The Journal of nutrition 131, 2636-2642.
Cocco, T., Sgobbo, P., Clemente, M., Lopriore, B., Grattagliano, I., Di Paola, M., Villani, G., 2005. Tissue-specific changes of mitochondrial functions in aged rats: effect of a long-term dietary treatment with N-acetylcysteine. Free Radic Biol Med 38, 796-805.
Darnold, J.R., Vorbeck, M.L., Martin, A.P., 1990. Effect of aging on the oxidative phosphorylation pathway. Mech Ageing Dev 53, 157-167.
Davis, L.M., Rho, J.M., Sullivan, P.G., 2008. UCP-mediated free fatty acid uncoupling of isolated cortical mitochondria from fasted animals: correlations to dietary modulations. Epilepsia 49 Suppl 8, 117-119.
Ferreira, I.L., Resende, R., Ferreiro, E., Rego, A.C., Pereira, C.F., 2010. Multiple defects in energy metabolism in Alzheimer's disease. Curr Drug Targets 11, 1193-1206.
Gnaiger, E., 2009. Capacity of oxidative phosphorylation in human skeletal muscle: new perspectives of mitochondrial physiology. Int J Biochem Cell Biol 41, 1837-1845.
Gnaiger, E., Kuznetsov, A.V., Lassnig, B., Fuchs, A., Reck, M., 1998. High-resolution respirometry - optimum permeabilization of the cell membrane by digitonin, in: Larsson, C., Påhlman, I.-L., Gustafsson, L. (Eds.), BioThermoKinetics in the Post Genomic Era, Chalmers Reproservice, Göteborg, pp. 85-95.
Gnaiger, E., Kuznetsov, A.V., Schneeberger, S., Seiler, R., Brandacher, G., Steurer, W., Margreiter, R., 2000. Mitochondria in the cold., in: Heldmaier, G., Klingenspor, M. (Eds.), Life in the Cold. Springer, Heidelberg, Berlin, New York, pp. 431-442.
Haas, R.H., Parikh, S., Falk, M.J., Saneto, R.P., Wolf, N.I., Darin, N., Wong, L.J., Cohen, B.H., Naviaux, R.K., 2008. The in-depth evaluation of suspected mitochondrial disease. Mol Genet Metab 94, 16-37.
Hauptmann, S., Keil, U., Scherping, I., Bonert, A., Eckert, A., Muller, W.E., 2006. Mitochondrial dysfunction in sporadic and genetic Alzheimer's disease. Experimental gerontology 41, 668-673.
Hutter, E., Unterluggauer, H., Garedew, A., Jansen-Durr, P., Gnaiger, E., 2006. High-resolution respirometry--a modern tool in aging research. Experimental gerontology 41, 103-109.
Johannsen, D.L., Ravussin, E., 2010. Obesity in the elderly: is faulty metabolism to blame? Aging health 6, 159-167.
Kilkson, H., Holme, S., Murphy, S., 1984. Platelet metabolism during storage of platelet concentrates at 22 degrees C. Blood 64, 406-414.
Kitchens, C.S., Newcomb, T.F., 1968. Human platelet respiration. J Appl Physiol 25, 581-585.
Kones, R., 2010. Parkinson's disease: mitochondrial molecular pathology, inflammation, statins, and therapeutic neuroprotective nutrition. Nutr Clin Pract 25, 371-389.
Krige, D., Carroll, M.T., Cooper, J.M., Marsden, C.D., Schapira, A.H., 1992. Platelet mitochondrial function in Parkinson's disease. The Royal Kings and Queens Parkinson Disease Research Group. Ann Neurol 32, 782-788.
Kuznetsov, A.V., Strobl, D., Ruttmann, E., Konigsrainer, A., Margreiter, R., Gnaiger, E., 2002. Evaluation of mitochondrial respiratory function in small biopsies of liver. Anal Biochem 305, 186-194.
Lemieux, H., Semsroth, S., Antretter, H., Hofer, D., Gnaiger, E., 2011. Mitochondrial respiratory control and early defects of oxidative phosphorylation in the failing human heart. Int J Biochem Cell Biol 43, 1729-1738.
Lenaz, G., Genova, M.L., 2009. Structural and functional organization of the mitochondrial respiratory chain: a dynamic super-assembly. Int J Biochem Cell Biol 41, 1750-1772.
Lesnefsky, E.J., Hoppel, C.L., 2006. Oxidative phosphorylation and aging. Ageing research reviews 5, 402-433.
Merlo Pich, M., Bovina, C., Formiggini, G., Cometti, G.G., Ghelli, A., Parenti Castelli, G., Genova, M.L., Marchetti, M., Semeraro, S., Lenaz, G., 1996. Inhibitor sensitivity of respiratory complex I in human platelets: a possible biomarker of ageing. FEBS Lett 380, 176-178.
Nicholls, D.G., 1977. The effective proton conductance of the inner membrane of mitochondria from brown adipose tissue. Dependency on proton electrochemical potential gradient. Eur J Biochem 77, 349-356.
Nulton-Persson, A.C., Szweda, L.I., 2001. Modulation of mitochondrial function by hydrogen peroxide. The Journal of biological chemistry 276, 23357-23361.
Picard, M., Taivassalo, T., Gouspillou, G., Hepple, R.T., 2011. Mitochondria: Isolation, Structure and Function. J Physiol.
Rasmussen, U.F., Krustrup, P., Kjaer, M., Rasmussen, H.N., 2003a. Experimental evidence against the mitochondrial theory of aging. A study of isolated human skeletal muscle mitochondria. Experimental gerontology 38, 877-886.
Rasmussen, U.F., Krustrup, P., Kjaer, M., Rasmussen, H.N., 2003b. Human skeletal muscle mitochondrial metabolism in youth and senescence: no signs of functional changes in ATP formation and mitochondrial oxidative capacity. Pflugers Archiv : European journal of physiology 446, 270-278.
Rosenstock, T.R., Duarte, A.I., Rego, A.C., 2010. Mitochondrial-associated metabolic changes and neurodegeneration in Huntington's disease - from clinical features to the bench. Curr Drug Targets 11, 1218-1236.
Rossignol, R., Faustin, B., Rocher, C., Malgat, M., Mazat, J.P., Letellier, T., 2003. Mitochondrial threshold effects. Biochem J 370, 751-762.
Sjövall, F., Morota, S., Hansson, M.J., Friberg, H., Gnaiger, E., Elmer, E., 2010. Sepsis induces platelet mitochondrial uncoupling and a gradual increase in respiratory capacity that is negatively associated with clinical outcome. Critical Care 14, P:11.
Tonkonogi, M., Fernstrom, M., Walsh, B., Ji, L.L., Rooyackers, O., Hammarqvist, F., Wernerman, J., Sahlin, K., 2003. Reduced oxidative power but unchanged antioxidative capacity in skeletal muscle from aged humans. Pflugers Archiv : European journal of physiology 446, 261-269.
Vendelbo, M.H., Nair, K.S., 2011. Mitochondrial longevity pathways. Biochimica et biophysica acta 1813, 634-644.
Xu, J., Shi, C., Li, Q., Wu, J., Forster, E.L., Yew, D.T., 2007. Mitochondrial dysfunction in platelets and hippocampi of senescence-accelerated mice. J Bioenerg Biomembr 39, 195-202.
Yamori, Y., 2006. Food factors for atherosclerosis prevention: Asian perspective derived from analyses of worldwide dietary biomarkers. Experimental and clinical cardiology 11, 94-98.
Yanicostas, C., Soussi-Yanicostas, N., El-Khoury, R., Benit, P., Rustin, P., 2011. Developmental aspects of respiratory chain from fetus to infancy. Seminars in fetal & neonatal medicine 16, 175-180.

## Claims

1. A method for screening and selection of potential drug candidates or for testing a person's sensitivity to a substance with effect on mitochondria, the method comprising
i) subjecting a sample of human blood cells containing mitochondria to high-resolution respirometry
ii) adding to the cell sample a substance that increases the leakage of the inner mitochondrial membrane to protons,
iii) adding to the sample from ii) a test substance in a vehicle,
vi) adding to the sample from iii) an inhibitor of mitochondrial complex I-function,
v) adding to the sample from vi) an inhibitor of mitochondrial complex III-function, and
vi) comparing the mitochondrial oxygen consumption before and after addition of the test substance with the mitochondrial oxygen consumption of the vehicle, wherein a decrease in mitochondrial oxygen consumption induced by the test substance indicates a negative effect on the mitochondria, and wherein an increase in mitochondrial oxygen consumption induced by the test substance indicates a positive effect on mitochondria.

2. A method for investigating mitochondrial effects of drug candidates in clinical trials or in treatment regimens, the method comprising
i) subjecting a sample of human blood cells containing mitochondria to high-resolution respirometry, wherein the sample of cells is from a person subjected to a clinical study or to a treatment regimen, and wherein a test substance has been administered to the person during the clinical study or treatment regimen
ii) adding to the cell sample a substance that increases the leakage of the inner mitochondrial membrane to protons,
iii) adding to the sample from ii) an inhibitor of mitochondrial complex I-function,
vi) adding to the sample from iii) an inhibitor of mitochondrial complex III-function, and
v) comparing the mitochondrial oxygen consumption of the sample from the person subjected to the clinical study or to a treatment regimen with the mitochondrial oxygen consumption of a control sample, wherein a decrease in mitochondrial oxygen consumption indicates a negative effect on the mitochondria, and wherein an increase in mitochondrial oxygen consumption indicates a positive effect on the mitochondria.

3. A method according to any of the preceding claims, wherein the substance that increases the leakage of the inner mitochondria membrane to protons is added to obtain maximal capacity of the electron transport system of the mitochondria present in the sample.

4. A method according to any of the preceding claims, wherein the substance that increases the leakage of the inner mitochondria membrane to protons is selected from carbonyl cyanide p-(trifluoromethoxy) phenylhydrazone (FCCP), carbonyl cyanide m-chlorophenylhydrazone (CCCP), 2,4-Dinitrophenol (DNP) and other protonophores, and mixtures thereof.

5. A method according to any of the preceding claims, wherein the substance that increases the leakage of the inner mitochondria membrane to protons is FCCP.

6. A method according to any of claims 1, 3-5, wherein the test sample is in liquid form and the test sample contains a known concentration of the test substance.

7. A method according to claim 6, wherein the test sample is added in stepwise increasing concentrations.

8. A method according to any of claims 1, 3-7, wherein the control sample in iii) is identical to the test sample but without content of test substance.

9. A method according to any of the preceding claims, wherein the inhibitor of mitochondrial complex I-function is added to elucidate the cellular respiration dependent on oxidation of complex II-substrates.

10. A method according to any of the preceding claims, wherein the inhibitor of mitochondrial complex I-function is rotenone.

11. A method according to any of the preceding claims, wherein the inhibitor of mitochondrial complex III-function is added to determine any non-mitochondrial oxygen-consuming activity, such as auto-oxidation of said sample.

12. A method according to any of the preceding claims, wherein the inhibitor of mitochondrial complex III-function is antimycin A.

13. A method according to any of claims 2-12, wherein the control sample is from a control group.

14. A method according to any of claims 2-13, wherein the control sample was taken before the start of any treatment.

15. A method according to any of claims 1, 3-12 for screening of drug candidates.

16. A method according to any of claims 1-14 for testing a subject's sensitivity to a drug substance or a drug candidate.

17. A method according to any of claims 2-14 for evaluating mitochondrial toxicity of a test substance in clinical trials, or for analysing the effect of a substance on mitochondrial function, or for evaluating compound capable of stimulating mitochondrial respiration and ATP production.

18. A method according to claim 16, wherein the sample of cells is isolated from said subject.

19. A method for screening and selecting potential drug candidates or for testing the sensitivity of a person to a substance with effect on mitochondria, the method comprising
i) subjecting a sample of human blood cells containing mitochondria from a human blood sample to high-resolution respirometry,
ii) adding to the sample of cell a substance that inhibits complex I respiration,
iii) adding to the sample of ii) a test substance in a vehicle, wherein an increase in respiration indicates plasma membrane permeability,
iv) adding to the sample of iii) a substance that permeabilizes the plasma membrane,
v) adding a reference substance to the sample obtained in iv) wherein the reference substance is a complex II-linked substrate, and
vi) adding to the sample of v) a substance that inhibits complex III respiration, and comparing the oxygen consumption obtained with the oxygen consumption obtained when the method is carried out using the reference substance in step v) and thus omitting addition of a test sample,
wherein the test substance permeates the plasma membrane if the oxygen consumption after addition of the test substance in iii) is higher than the oxygen consumption after addition of the reference substance in iii), when the method is carried out using the reference substance, and
wherein non-mitochondrial oxygen consumption in induced by the test substance if the oxygen consumption after addition of a substance that inhibits complex III respiration in v) is higher than the oxygen consumption in v), when the method is carried out using the reference substance.

20. A method according to claim 19, wherein the substance that permeabilizes the plasma membrane is digitonin.

21. A method according to any of claims 19-20, wherein the reference substance is succinate.

22. A method according to any of claims 19-21, wherein the details for the individual steps i) - vi) are as defined in any one of claims 6, 7, 9, 10, 11, 12, 15, and/or 17.

## Patentansprüche

1. Ein Verfahren zum Screening und Auswählen eines potentiellen Arzneimittelkandidaten oder zum Testen der Sensitivität einer Person auf eine Substanz mit Wirkung auf Mitochondrien, das Verfahren umfassend
i) Unterziehen einer Probe menschlicher Blutzellen, die Mitochondrien enthalten, einer hochauflösenden Respirometrie,
ii) Zugeben zur Zellprobe einer Substanz, die die Permeabilität der inneren Mitochondrienmembran für Protonen erhöht,
iii) Zugeben zur Probe aus ii) einer Testsubstanz in einem Vehikel,
iv) Zugeben zur Probe aus iii) eines Inhibitors der mitochondrialen Komplex-I-Funktion,
v) Zugeben zur Probe aus iv) eines Inhibitors der mitochondrialen Komplex-III-Funktion,
und
vi) Vergleichen des mitochondrialen Sauerstoffverbrauchs vor und nach Zugabe der Testsubstanz mit dem mitochondrialen Sauerstoffverbrauch des Vehikels, wobei eine durch die Testsubstanz induzierte Abnahme des Sauerstoffverbrauchs eine negative Wirkung auf die Mitochondrien indiziert, und wobei eine durch die Testsubstanz induzierte Steigerung des Sauerstoffverbrauchs eine positive Wirkung auf die Mitochondrien indiziert.

2. Ein Verfahren zur Untersuchung mitochondrialer Wirkungen von Arzneimittelkandidaten in klinischen Studien oder in Behandlungsregimen, das Verfahren umfassend
i) Unterziehen einer Probe menschlicher Blutzellen, die Mitochondrien enthalten, einer hochauflösenden Respirometrie, wobei die Zellprobe von einer Person stammt, die sich einer klinischen Studie oder einem Behandlungsregime unterzieht, und wobei eine Testsubstanz der Person während der klinischen Studie oder dem Behandlungsregime verabreicht wurde,
ii) Zugeben zur Zellprobe einer Substanz, die die Permeabilität der inneren Mitochondrienmembran für Protonen erhöht,
iii) Zugeben zur Probe aus ii) eines Inhibitors der mitochondrialen Komplex-I-Funktion,
iv) Zugeben zur Probe aus iii) eines Inhibitors der mitochondrialen Komplex-III-Funktion,
und
Vergleichen des mitochondrialen Sauerstoffverbrauchs der Probe von der Person, die sich einer klinischen Studie oder einem Behandlungsregime unterzog mit dem mitochondrialen Sauerstoffverbrauch einer Kontrollprobe, wobei eine Abnahme des Sauerstoffverbrauchs eine negative Wirkung auf die Mitochondrien indiziert, und wobei eine Steigerung des Sauerstoffverbrauchs eine positive Wirkung auf die Mitochondrien indiziert.

3. Verfahren nach einem der vorhergehenden Ansprüche, wobei die Substanz, die die Permeabilität der inneren Mitochondrienmembran gegenüber Protonen erhöht, zugegeben wird, um eine maximale Kapazität des Elektronentransportsystems der in der Probe vorhandenen Mitochondrien zu erhalten.

4. Verfahren nach einem der vorhergehenden Ansprüche, wobei die Substanz, die die Permeabilität der inneren Mitochondrienmembran für Protonen erhöht, ausgewählt ist aus Carbonylcyanid-p-(trifluormethoxy)phenylhydrazon (FCCP), Carbonylcyanid-m-chlorphenylhydrazon (CCCP), 2,4-Dinitrophenol (DNP) und andere Protonophore und Mischungen davon.

5. Verfahren nach einem der vorhergehenden Ansprüche, wobei die Substanz, die die Permeabilität der inneren Mitochondrienmembran für Protonen erhöht, FCCP ist.

6. Verfahren nach einem der Ansprüche 1, 3-5, wobei die Testprobe in flüssiger Form vorliegt und die Testprobe eine bekannte Konzentration der Testsubstanz enthält.

7. Verfahren nach Anspruch 6, wobei die Testprobe in stufenweise ansteigenden Konzentrationen zugegeben wird.

8. Verfahren nach einem der Ansprüche 1, 3-7, wobei die Kontrollprobe in iii) identisch mit der Testprobe ist, jedoch ohne Gehalt an Testsubstanz.

9. Verfahren nach einem der vorhergehenden Ansprüche, wobei der Inhibitor der mitochondrialen Komplex-I-Funktion zugegeben wird, um die zelluläre Atmung in Abhängigkeit von der Oxidation von Komplex-II-Substraten aufzuklären.

10. Verfahren nach einem der vorhergehenden Ansprüche, wobei der Inhibitor der mitochondrialen Komplex-I-Funktion Rotenon ist.

11. Verfahren nach einem der vorangehenden Ansprüche, wobei der Inhibitor der mitochondrialen Komplex-III-Funktion zugegeben wird, um jegliche nicht mitochondriale Sauerstoff verbrauchende Aktivität, wie die Autooxidation der Probe, zu bestimmen.

12. Verfahren nach einem der vorhergehenden Ansprüche, wobei der Inhibitor der mitochondrialen Komplex-III-Funktion Antimycin A ist.

13. Verfahren nach einem der Ansprüche 2-12, wobei die Kontrollprobe von einer Kontrollgruppe stammt.

14. Verfahren nach einem der Ansprüche 2-13, wobei die Kontrollprobe vor Beginn einer Behandlung entnommen wurde.

15. Verfahren nach einem der Ansprüche 1, 3-12 zum Screening von Arzneimittelkandidaten.

16. Verfahren nach einem der Ansprüche 1-14 zum Testen der Sensitivität einer Person auf eine Arzneimittelsubstanz oder einen Arzneimittelkandidaten.

17. Verfahren nach einem der Ansprüche 2-14 zum Bewerten der mitochondrialen Toxizität einer Testsubstanz in klinischen Studien oder zum Analysieren der Wirkung einer Substanz auf die mitochondriale Funktion oder zum Bewerten der Fähigkeit einer Substanz die mitochondriale Atmung und ATP-Produktion zu stimulieren.

18. Verfahren nach Anspruch 16, wobei die Zellprobe von jenem Subjekt isoliert wird.

19. Ein Verfahren zum Screening und Auswählen eines potentiellen Arzneimittelkandidaten oder zum Testen der Sensitivität einer Person auf eine Substanz mit Wirkung auf Mitochondrien, das Verfahren umfassend
i) Unterziehen einer Probe menschlicher Blutzellen, die Mitochondrien aus einer menschlichen Blutprobe enthalten, einer hochauflösenden Respirometrie,
ii) Zugeben zur Zellprobe einer Substanz, die die Komplex-I-Atmung inhibiert,
iii) Zugeben zur Probe aus ii) einer Testsubstanz in einem Vehikel, wobei eine Steigerung der Atmung die Plasmamembranpermeabilität indiziert,
iv) Zugeben zur Zellprobe aus iii) einer Substanz, die die Plasmamembran permeabilisiert,
v) Zugeben einer Referenzsubstanz zu der in iv) erhaltenen Probe, wobei die Referenzsubstanz ein Komplex-II-gekoppeltes Substrat ist, und
vi) Zugeben zur Probe aus v) einer Substanz, die die Komplex-III-Atmung inhibiert, und
Vergleichen des Sauerstoffverbrauchs mit dem Sauerstoff Verbrauch, der erhalten wird, wenn das Verfahren unter Verwendung der Referenzsubstanz in Schritt v) durchgeführt wird und somit die Zugabe einer Testprobe weggelassen wird, wobei die Testsubstanz, die Plasmamembran permeabilisiert, wenn der Sauerstoffverbrauch nach Zugabe der Testsubstanz in iii) höher ist als der Sauerstoffverbrauch nach Zugabe der Referenzsubstanz in iii), wenn das Verfahren unter Verwendung der Referenzsubstanz durchgeführt wird, und wobei der nicht-mitochondriale Sauerstoffverbrauch durch die Testsubstanz induziert wird, wenn der Sauerstoffverbrauch nach Zugabe einer Substanz, die die Komplex-III-Atmung inhibiert, in Schritt v) höher ist als der Sauerstoffverbrauch in v), wenn das Verfahren unter Verwendung der Referenzsubstanz durchgeführt wird.

20. Verfahren nach Anspruch 19, wobei die Substanz, die die Plasmamembran permeabilisiert, Digitonin ist.

21. Verfahren nach einem der Ansprüche 19-20, wobei die Referenzsubstanz Succinat ist.

22. Verfahren nach einem der Ansprüche 19-21, wobei die Details für die einzelnen Schritte i) - vi) wie in einem der Ansprüche 6, 7, 9, 10, 11, 12, 15 und/oder 17 definiert sind.

## Revendications

1. Méthode de dépistage et de sélection de médicaments candidats potentiels ou d'analyse d'une sensibilité d'une personne vis-à-vis d'une substance ayant un effet sur les mitochondries, la méthode comprenant
i) la soumission d'un échantillon de cellules sanguines humaines contenant des mitochondries à une respirométrie haute résolution,
ii) l'ajout à l'échantillon de cellules d'une substance qui augmente la fuite des protons à travers la membrane mitochondriale interne,
iii) l'ajout à l'échantillon de ii) d'une substance à tester dans un véhicule,
iv) l'ajout à l'échantillon de iii) d'un inhibiteur de la fonction mitochondriale du complexe I,
v) l'ajout à l'échantillon de iv) d'un inhibiteur de la fonction mitochondriale du complexe III, et
vi) la comparaison de la consommation d'oxygène des mitochondries avant et après l'ajout de la substance à tester avec la consommation d'oxygène des mitochondries dans le véhicule, une diminution de la consommation d'oxygène des mitochondries induite par la substance à tester indiquant un effet négatif sur les mitochondries et une augmentation de la consommation d'oxygène des mitochondries induite par la substance à tester indiquant un effet positif sur les mitochondries.

2. Méthode d'étude des effets mitochondriaux des médicaments candidats dans des essais cliniques ou dans des schémas de traitement, la méthode comprenant
i) la soumission d'un échantillon de cellules sanguines humaines contenant des mitochondries à une respirométrie haute résolution, l'échantillon de cellules provenant d'une personne soumise à une étude clinique ou à un schéma de traitement, et une substance à tester ayant été administrée à la personne pendant l'étude clinique ou le schéma de traitement,
ii) l'ajout à l'échantillon de cellules d'une substance qui augmente la fuite des protons à travers la membrane mitochondriale interne,
iii) l'ajout à l'échantillon de ii) d'un inhibiteur de la fonction mitochondriale du complexe I,
iv) l'ajout à l'échantillon de iii) d'un inhibiteur de la fonction mitochondriale du complexe III, et
v) la comparaison de la consommation d'oxygène des mitochondries de la personne soumise à une étude clinique ou à un schéma de traitement avec la consommation d'oxygène des mitochondries d'un échantillon témoin, une diminution de la consommation d'oxygène des mitochondries indiquant un effet négatif sur les mitochondries et une augmentation de la consommation d'oxygène des mitochondries indiquant un effet positif sur les mitochondries.

3. Méthode selon l'une quelconque des revendications précédentes, dans laquelle la substance qui augmente la fuite des protons à travers la membrane mitochondriale interne est ajoutée pour obtenir la capacité maximale du système de transport d'électrons des mitochondries présentes dans l'échantillon.

4. Méthode selon l'une quelconque des revendications précédentes, dans laquelle la substance qui augmente la fuite des protons à travers la membrane mitochondriale interne est choisie parmi la carbonylcyanure p-(trifluorométhoxy)phényl-hydrazone (FCCP), la carbonylcyanure m-chlorophénylhydrazone (CCCP), le 2,4-dinitrophénol (DNP) et d'autres protonophores, et des mélanges de ceux-ci.

5. Méthode selon l'une quelconque des revendications précédentes, dans laquelle la substance qui augmente la fuite des protons à travers la membrane mitochondriale interne est la FCCP.

6. Méthode selon l'une quelconque des revendications 1, 3 à 5, dans laquelle l'échantillon à tester est sous une forme liquide et l'échantillon à tester contient une concentration connue de la substance à tester.

7. Méthode selon la revendication 6, dans laquelle l'échantillon à tester est ajouté selon des concentrations augmentant par étape.

8. Méthode selon l'une quelconque des revendications 1, 3 à 7, dans laquelle l'échantillon témoin de iii) est identique à l'échantillon à tester, mais sans substance à tester.

9. Méthode selon l'une quelconque des revendications précédentes, dans laquelle l'inhibiteur de la fonction mitochondriale du complexe I est ajouté pour élucider la respiration cellulaire qui est dépendante de l'oxydation de substrats du complexe II.

10. Méthode selon l'une quelconque des revendications précédentes, dans laquelle l'inhibiteur de la fonction mitochondriale du complexe I est la roténone.

11. Méthode selon l'une quelconque des revendications précédentes, dans laquelle l'inhibiteur de la fonction mitochondriale du complexe III est ajouté pour déterminer une quelconque activité de consommation d'oxygène non mitochondriale, telle que l'auto-oxydation dudit échantillon.

12. Méthode selon l'une quelconque des revendications précédentes, dans laquelle l'inhibiteur de la fonction mitochondriale du complexe III est l'antimycine A.

13. Méthode selon l'une quelconque des revendications 2 à 12, dans laquelle l'échantillon témoin provient d'un groupe témoin.

14. Méthode selon l'une quelconque des revendications 2 à 13, dans laquelle l'échantillon témoin a été prélevé avant le début d'un quelconque traitement.

15. Méthode selon l'une quelconque des revendications 1, 3 à 12, pour le dépistage de médicaments candidats.

16. Méthode selon l'une quelconque des revendications 1 à 14, pour tester la sensibilité d'un sujet vis-à-vis d'une substance médicamenteuse ou d'un médicament candidat.

17. Méthode selon l'une quelconque des revendications 2 à 14, pour l'évaluation de la toxicité mitochondriale d'une substance à tester dans des essais cliniques, ou pour l'analyse de l'effet d'une substance sur la fonction mitochondriale ou pour l'évaluation d'un composé capable de stimuler la respiration mitochondriale et la production d'ATP.

18. Méthode selon la revendication 16, dans laquelle l'échantillon de cellules est isolé auprès dudit sujet.

19. Méthode de dépistage et de sélection de médicaments candidats potentiels ou d'analyse de la sensibilité d'une personne vis-à-vis d'une substance ayant un effet sur les mitochondries, la méthode comprenant
i) la soumission d'un échantillon de cellules sanguines humaines contenant des mitochondries provenant d'un échantillon de sang humain à une respirométrie haute résolution,
ii) l'ajout à l'échantillon de cellules d'une substance qui inhibe la respiration du complexe I,
iii) l'ajout à l'échantillon de ii) d'une substance à tester dans un véhicule, une augmentation de la respiration indiquant la perméabilité de la membrane plasmatique,
iv) l'ajout à l'échantillon de iii) d'une substance qui perméabilise la membrane plasmatique,
v) l'ajout d'une substance de référence à l'échantillon obtenu en iv), la substance de référence étant un substrat lié au complexe II, et
vi) l'ajout à l'échantillon de v) d'une substance qui inhibe la respiration du complexe III, et la comparaison de la consommation d'oxygène obtenue avec la consommation d'oxygène obtenue lorsque la méthode est réalisée en utilisant la substance de référence de l'étape v) et en omettant donc l'ajout d'un échantillon à tester,
la substance à tester s'infiltrant dans la membrane plasmatique si la consommation d'oxygène après l'ajout de la substance à tester en iii) est supérieure à la consommation d'oxygène après l'ajout de la substance de référence en iii), lorsque la méthode est réalisée en utilisant la substance de référence, et
la consommation d'oxygène non mitochondriale étant induite par la substance à tester si la consommation d'oxygène après l'ajout d'une substance qui inhibe la respiration du complexe III en v) est supérieure à la consommation d'oxygène en v) lorsque la méthode est réalisée en utilisant la substance de référence.

20. Méthode selon la revendication 19, dans laquelle la substance qui perméabilise la membrane plasmatique est la digitonine.

21. Méthode selon l'une quelconque des revendications 19 à 20, dans laquelle la substance de référence est le succinate.

22. Méthode selon l'une quelconque des revendications 19 à 21, dans laquelle les détails pour les étapes individuelles i) à vi) sont tels que définis selon l'une quelconque des revendications 6, 7, 9, 10, 11, 12, 15 et/ou 17.
